# EUROPEAN PATENT SPECIFICATION

(11) **EP 4 413 912 B1**
(45) Date of publication and mention of the grant of the patent: **13.05.2026**
(21) Application number: 24154114.3
(22) Date of filing: 26.01.2024
(51) Int. Cl.: A61B 1/00, A61B 1/005

(54) **ENDOSCOPE**
ENDOSKOP
ENDOSCOPE

(30) Priority: 10.02.2023 US 202363444640 P
(43) Date of publication of application: 14.08.2024
(73) Proprietor: Olympus Medical Systems Corp., Tokyo 192-8507 (JP)
(72) Inventor: SUMI, Takahiro, Tokyo, 192-8507 (JP); KATO, Akihiro, Tokyo, 192-8507 (JP)
(74) Representative: Schicker, Silvia

(56) References cited:
- WO-A1-2021/193695

## Description

### BACKGROUND OF THE INVENTION

### 1. Field of the Invention

The invention relates to an endoscope including, in an operation section, an operation lever as an operation member that operates a movable member provided in a distal end portion.

### 2. Description of Related Art

Conventionally, endoscopes have been widely utilized in the medical field and the industrial field, for example. A medical endoscope used in the medical field is configured to include an insertion section formed into an elongated tubular shape and having an image pickup unit and the like in a distal end portion, and an operation section provided consecutively to a proximal end side of the insertion section and having various operation members and the like on an outer surface.

Such a conventional endoscope has a function of inserting the insertion section into a body cavity of a living body to acquire, display, and record internal images of an organ or the like. A user observes and inspects a site of lesion or the like in an organ or the like based on images acquired and displayed by the endoscope.

In some cases, such a conventional endoscope may be configured to include a conduit (called a treatment instrument insertion conduit) that allows insertion of a treatment instrument or the like in an insertion section, a forceps raising base (forceps elevator) that changes a protruding direction of the treatment instrument in a state allowed to be inserted through the conduit and caused to protrude to the outside of a distal end portion of the insertion section, and the like. Herein, the treatment instrument is an instrument including a forceps and the like at a distal end and used for performing various types of treatment such as a biopsy of collecting some living tissues in a body cavity and resection of a site of lesion in the body cavity. The forceps raising base has a structure to be driven in accordance with operation of a forceps lever which is a predetermined operation member and an operation lever provided in the operation section.

An endoscope disclosed by Japanese Patent Application Laid-Open Publication No. 2020-89598 or Japanese Patent Application Laid-Open Publication No. 2011-136193, for example, has a form in which a forceps raising base is provided in a distal end portion of an insertion section of the endoscope. In the endoscope, an operation lever which is an operation member for operating the forceps raising base is provided in an operation section. A driving mechanism including a traction wire for driving the forceps raising base in accordance with operation of the operation lever is provided between the forceps raising base and the operation lever.

Some conventional endoscopes include a bending portion obtained by configuring a portion of an insertion section to be bendable in an insertion axis direction. The bending portion in such a conventional endoscope has a configuration to be driven in a bending manner in accordance with operation of an operation lever which is a predetermined operation member provided in an operation section.

An endoscope disclosed by Japanese Patent Application Laid-Open Publication No. 2005-13320, for example, includes a bending portion obtained by configuring a partial region of an insertion section closer to a distal end to be bendable. An operation lever which is an operation member that performs a bending operation of the bending portion is provided in an operation section. A driving mechanism including a traction wire for driving the bending portion in a bending manner in accordance with operation of the operation lever is provided between the bending portion and the operation lever.

Some other conventional endoscopes include a configuration in which an image pickup visual field and an image pickup magnification can be changed within predetermined ranges by moving in an optical axis direction some optical members of an image pickup optical system included in an image pickup unit provided in a distal end portion of an insertion section. The image pickup optical system in such a conventional endoscope has a configuration in which some optical members move in the optical axis direction in accordance with operation of an operation lever which is a predetermined operation member provided in an operation section. In this case, a driving mechanism including a traction wire for causing some optical members of the image pickup optical system to move forward and backward in accordance with operation of the operation lever is provided between the image pickup unit and the operation lever.

As described above, among conventional endoscopes, various endoscopes having a configuration of driving and operating various movable members (such as a forceps raising base, a bending portion, and a portion of an image pickup optical system) provided in a distal end portion of an insertion section or an area closer to a distal end of the insertion section in accordance with operation of an operation lever which is a predetermined operation member provided in an operation section have been put to practical use.

Such a conventional endoscope is commonly provided with a structure for restricting an operation range of the operation lever which is the operation member to a predetermined range.

The endoscope disclosed by Japanese Patent Application Laid-Open Publication No. 2020-89598 described earlier, for example, is configured to have a transmission member, a moving member, and a stop member. The transmission member transmits an operation input of the operation lever to the moving member. The moving member moves in a predetermined direction of action in accordance with an amount of force transmitted through the transmission member (that is, in accordance with the operation input of the operation lever). Herein, the moving member has one end coupled to the traction wire and the other end coupled to the transmission member. The stop member is fixed to the operation section. The endoscope has a structure in which movement of the moving member is restricted when a portion (an abutting surface) of the moving member comes into contact with a portion (a surface opposite to the abutting surface) of the stop member.

The endoscope disclosed by Japanese Patent Application Laid-Open Publication No. 2011-136193 described earlier or the like is configured to have the operation lever and a stopper member that restricts an operation range of the forceps raising base. The endoscope disclosed by Japanese Patent Application Laid-Open Publication No. 2005-13320 described earlier or the like is configured to have the operation lever and a stopper member that restricts a bending operation range of the bending portion.

Further, in the endoscope disclosed by Japanese Patent Application Laid-Open Publication No. 2011-136193 described earlier, Japanese Patent Application Laid-Open Publication No. 2005-13320 described earlier, or the like, the stopper member has a turning range adjustment mechanism that adjusts a turning range of the operation lever in a plurality of stages. The configuration including such a turning range adjustment mechanism enables a first state of intermediately stopping operation of the operation lever and a second state of stopping operation of the operation lever at a position where operation of the operation lever in the entire operation range is allowed to be selectively set.

However, the configurations of the conventional endoscopes disclosed by Japanese Patent Application Laid-Open Publication No. 2011-136193 described earlier, Japanese Patent Application Laid-Open Publication No. 2005-13320 described earlier, and the like may cause interference between an operation lever and another constituent member in a case where a user wants to set a stepwise operation range by a wider operation range. Therefore, such configurations raise a problem in that the operation range of the operation lever that can be set is limited.

For example, in a case of performing work, such as repair work for an endoscope, accompanied by disassembly work or the like, there is a request for ensuring a wider movable range of the operation lever in order to ensure efficient workability.

WO 2021/193695 A1 is the closest prior art disclosing the preamble of claim 1.

### SUMMARY OF THE INVENTION

The object of the present invention is to provide an endoscope that enables an appropriate operation range of an operation lever to be set during normal use and in a case of performing disassembly and repair work or the like, enables a wider movable range of the operation lever to be easily ensured with a simple configuration. In order to address this object, an endoscope having the features defined in claim 1 is provided. Preferred embodiments are defined by the dependent claims.

An endoscope according to the present invention includes an insertion section including a movable member, and an operation section including a lever and a stopper, and provided on a proximal end side of the insertion section. The movable member is configured to be movable in a first direction. The lever is held to be turnable around a first rotation axis, the lever being configured to turn to move the movable member. The stopper is configured to come into contact with the lever when the lever is located at a first position. Further, the lever is configured to be deformed between a first state and a second state. The first state is a state in which the lever comes into contact with the stopper at the first position. The second state is a state in which the lever does not come into contact with the stopper at the first position.

Hence, the present invention can provide an endoscope that enables an appropriate operation range of an operation lever to be set during normal use and in a case of performing disassembly and repair work or the like, enables a wider movable range of the operation lever to be easily ensured with a simple configuration.

The lever may include a projection. The projection preferably is formed in a second direction parallel to a direction in which the first rotation axis extends. The first state may be a state in which the projection comes into contact with the stopper. Alternatively or additionally, the second state may be a state in which the projection does not come into contact with the stopper.

In a preferred embodiment of the endoscope, the lever has a bearing which may be formed by joining a first hole and a second hole, the first rotation axis being inserted into the bearing. The first state may be a state in which the first rotation axis is inserted into the first hole and the lever is held to be turnable around the first rotation axis in the first hole. Alternatively or additionally, the second state may be a state in which the first rotation axis is inserted into the second hole and the lever is held to be turnable around the first rotation axis in the second hole.

The bearing may be formed by joining the first hole and the second hole into an arc shape.

The movable member may be a raising base which may be provided in the insertion section. The second rotation axis may be a rotation axis of the raising base.

The movable member may be at least one optical element on which light from a subject is incident.

The first position may be a position at which the movable member is restricted from moving to the proximal end side. Alternatively or additionally, the first position may be a position at which the movable member is restricted from moving to a distal end side.

In a preferred embodiment of the endoscope, the movable member and the lever are at least partially connected with a single-use member.

The operation section may include a link mechanism. Preferably, the link mechanism includes at least one of a link main body, a rod body, a coupling member, and a coupling member guide.

The rod body may have a first end coupled to the link main body. Alternatively or additionally, the rod body may have a second end coupled to the coupling member. The coupling member may be housed in the coupling member guide. When the link main body turns around the first rotation axis, the coupling member may be configured to slide inside the coupling member guide to move forward and backward in a long axis direction of the insertion section.

A portion of the first rotation axis may have a cross-section formed into a D-shape in a plane perpendicular to the second direction.

Preferred embodiments of the invention will now be described in greater detail with reference to the accompanying drawings

### BRIEF DESCRIPTION OF THE DRAWINGS

Fig. 1 is an external view showing a schematic configuration of an endoscope of a first embodiment not according to the present invention;
Fig. 2 is an external perspective view showing an area of an operation section of the endoscope as seen in a direction indicated by an arrow sign [2] in Fig. 1;
Fig. 3 is an external perspective view showing only a forceps lever in the endoscope in Fig. 1 having been taken out;
Fig. 4 is a plan view as seen in a direction indicated by an arrow sign [4] in Fig. 3;
Fig. 5 is a cross-sectional view along a line [5]-[5] in Fig. 4;
Fig. 6 is a cross-sectional view along a line [6]-[6] in Fig. 5;
Fig. 7 is an external perspective view showing only a finger rest member in the forceps lever in Fig. 3 having been taken out;
Fig. 8 is a cross-sectional view taken along a plane indicated by signs [8] in Fig. 7;
Fig. 9 is a diagram showing the cross-section in Fig. 8 as seen in a direction indicated by an arrow sign [9];
Fig. 10 is a diagram showing a turning range of the forceps lever in Fig. 3 during normal use;
Fig. 11 is a perspective view showing a schematic configuration including a forceps raising base which is a movable member provided in a distal end portion of an insertion section in the endoscope of the first embodiment;
Fig. 12 is a plan view showing a schematic configuration of a driving mechanism for the forceps lever in an internal configuration of the operation section in the endoscope of the first embodiment (when the forceps raising base is in a maximum inverted state);
Fig. 13 is a plan view showing a schematic configuration of the driving mechanism for the forceps lever in the internal configuration of the operation section in the endoscope of the first embodiment (when the forceps raising base is in a maximum raised state);
Fig. 14 is an essential part exploded perspective view showing some constituent members (a frame shaft and a link main body) of the driving mechanism for the forceps lever in Fig. 12 having been taken out;
Fig. 15 is an essential part magnified perspective view showing only a partial constituent member (a coupling member) of the driving mechanism for the forceps lever in Fig. 12 having been taken out;
Fig. 16 is an essential part magnified view showing under magnification a partial region (a neighboring region in a state in which the coupling member is in contact with a lock plate) of the driving mechanism for the forceps lever in Fig. 12;
Fig. 17 is a schematic perspective view showing a configuration of the forceps lever and a finger-rest-member removal jig in the endoscope of the first embodiment;
Fig. 18 is a partial cross-sectional view along a plane indicated by signs [18] in Fig. 17;
Fig. 19 is a schematic perspective view showing an action when detaching the finger rest member from the forceps lever in the endoscope of the first embodiment (a state in which a jig is inserted into a jig insertion groove of the finger rest member);
Fig. 20 is a partial cross-sectional view along a plane indicated by signs [20] in Fig. 19 (a state in which a lever lock portion is locked in a to-be-locked hole);
Fig. 21 is a partial cross-sectional view along the plane indicated by the signs [20] in Fig. 19 (a state in which a locked state of the lever lock portion in the to-be-locked hole is released);
Fig. 22 is a schematic perspective view showing an action when detaching the finger rest member from the forceps lever in the endoscope of the first embodiment (a state after the finger rest member is detached by the jig);
Fig. 23 is a partial cross-sectional view along a plane indicated by signs [23] in Fig. 22;
Fig. 24 is a diagram showing an action of the forceps lever from which the finger rest member has been detached in the endoscope of the first embodiment;
Fig. 25 is a diagram showing an action when attaching the removal jig to the lever member in which the finger rest member has been detached from the forceps lever (a state before attachment);
Fig. 26 is a diagram showing an action when attaching the removal jig to the lever member in which the finger rest member has been detached from the forceps lever (a state in which attachment is completed);
Fig. 27 is a diagram showing a first modification of the forceps lever in the endoscope of the first embodiment;
Fig. 28 is a diagram showing the operation section to which the forceps lever in Fig. 27 is applied;
Fig. 29 is a diagram showing a second modification of the forceps lever in the endoscope of the first embodiment;
Fig. 30 is a diagram showing a third modification of the endoscope of the first embodiment;
Fig. 31 is a perspective view showing only a lever member of a forceps lever in an endoscope of a second embodiment according to or of the present invention having been taken out;
Fig. 32 is an essential part exploded perspective view showing the forceps lever (the lever member) in Fig. 31 and some constituent members (the frame shaft and the link main body) in a driving mechanism for the forceps lever having been taken out;
Fig. 33 is a diagram showing an action of the forceps lever in Fig. 31 (a first state);
Fig. 34 is a diagram showing an action of the forceps lever in Fig. 31 (a second state);
Fig. 35 is a perspective view showing only a lever member of a forceps lever of a modification of the second embodiment of the present invention having been taken out;
Fig. 36 is an essential part exploded perspective view showing the forceps lever in Fig. 35 and some constituent members (the frame shaft and the link main body) in a driving mechanism for the forceps lever having been taken out;
Fig. 37 is a diagram showing an action of the forceps lever in the modification of the second embodiment of the present invention, showing the first state of the forceps lever;
Fig. 38 is a diagram showing an action of the forceps lever in the modification of the second embodiment of the present invention, showing the second state of the forceps lever;
Fig. 39 is a perspective view showing only a lever member of a forceps lever of a second modification in the endoscope of the second embodiment of the present invention having been taken out;
Fig. 40 is a perspective view showing only a lever member of a forceps lever of a third modification in the endoscope of the second embodiment of the present invention having been taken out; and
Fig. 41 is a diagram showing a modification of a movable member disposed in the distal end portion in the endoscope of the present invention.

### DETAILED DESCRIPTION OF PREFERRED EMBODIMENTS

Hereinafter, the present invention will be described by way of embodiments illustrated in the drawings. Each drawing used in the following description is schematically illustrated, and in order to illustrate each constituent element in a recognizable size on the drawing, a dimensional relationship, a scale, or the like of each member may be illustrated differently for each constituent element. Therefore, the present invention is not limited only to the embodiments illustrated in the drawings with respect to the number of each constituent element, the shape of each constituent element, the ratio of the size of each constituent element, the relative positional relationship of each constituent element, and the like illustrated in each drawing.

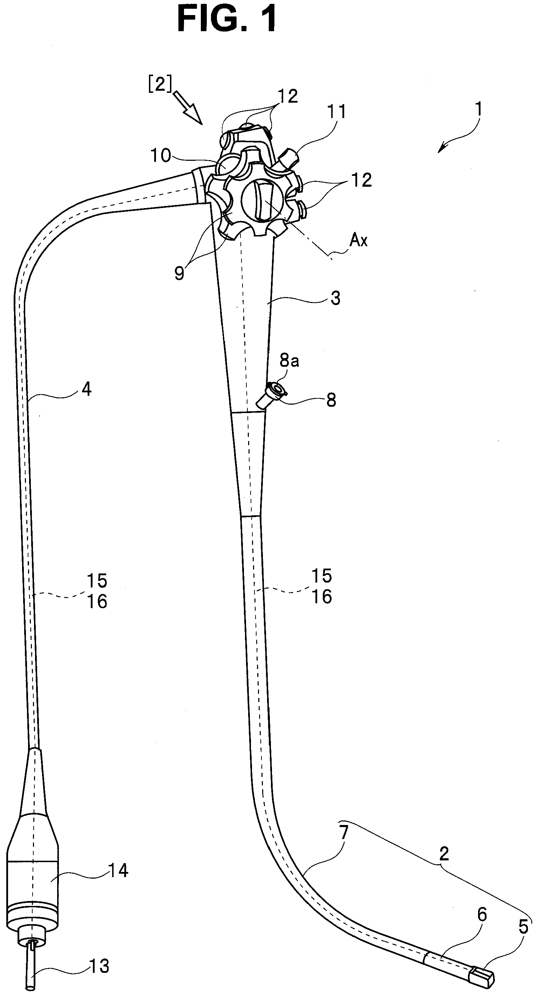

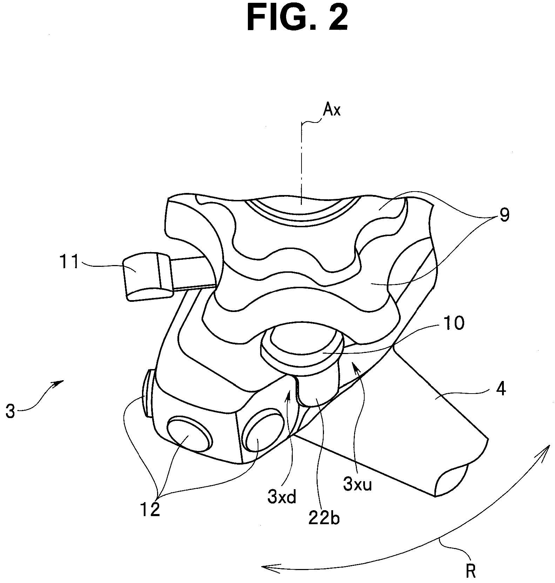

First, a schematic configuration of an endoscope of a first embodiment will be described below with reference to Fig. 1 and Fig. 2. Fig. 1 is an external view showing a schematic configuration of the endoscope of the first embodiment. Fig. 2 is an external perspective view showing an area of an operation section of the endoscope as seen in a direction indicated by an arrow sign [2] in Fig. 1.

The endoscope 1 is configured to include an insertion section 2, an operation section 3, a universal cable 4, and the like as shown in Fig. 1.

The insertion section 2 is a long member having an elongated tubular shape that may be inserted into a lumen of a subject such as a living body (the inside of an organ or the like). The insertion section 2 is configured to have a distal end portion 5, a bending portion 6, and a flexible tube portion 7.

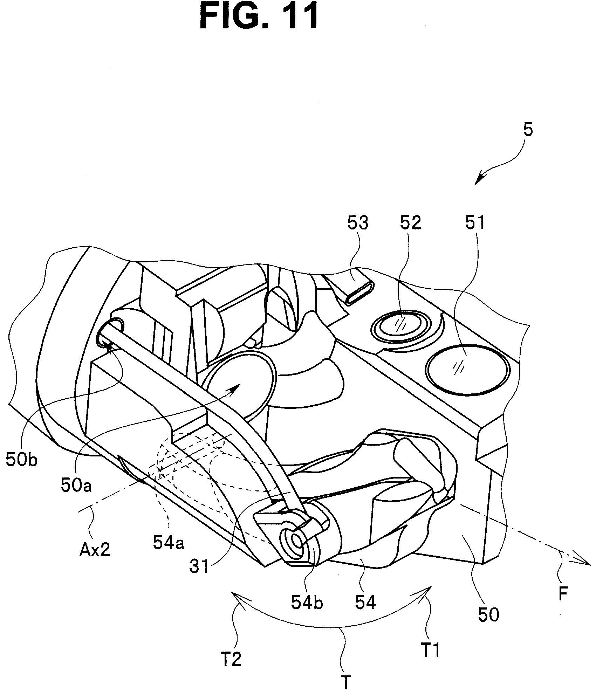

The distal end portion 5 is disposed at a distal end of the insertion section 2. Note that although illustration is omitted, a forceps raising base (not shown in Fig. 1; see reference numeral 54 in Fig. 11 which will be described later) which is a movable member, and the like are disposed in the distal end portion 5 in addition to an image pickup unit including an image pickup optical system, an image sensor, and the like, an illumination unit including an observation optical system and the like, a gas/liquid feeding nozzle and a gas/liquid feeding conduit, and the like.

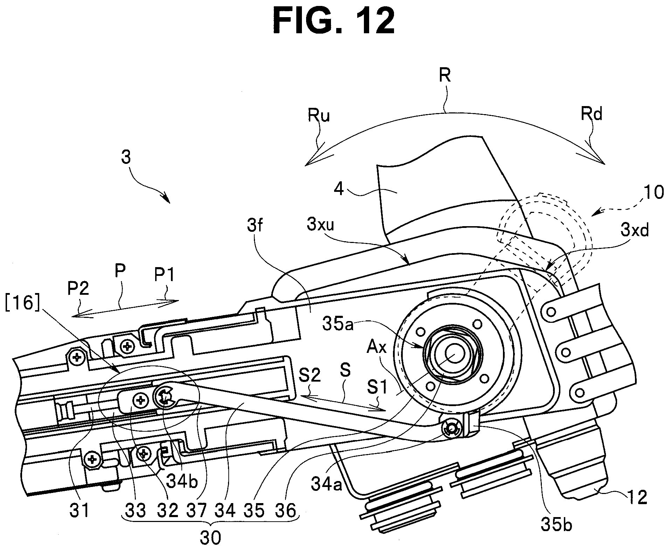

Herein, the forceps raising base is a constituent unit provided for changing a protruding direction of a treatment instrument (not shown) in a state allowed to be inserted through a treatment instrument insertion conduit (not shown) allowed to be inserted through and arranged in the insertion section 2 to be caused to protrude to the outside of the distal end portion 5. The forceps raising base is coupled to a forceps lever 10 (which will be described later) through a traction wire 31 allowed to be inserted and arranged between the insertion section 2 and the operation section 3 and a driving mechanism 30 (neither shown in Fig. 1; see Fig. 12 and the like which will be described later) provided inside the operation section 3. With this configuration, the forceps raising base functions as a movable member that moves in a predetermined direction in accordance with an operation input of the forceps lever 10 (which will be described later in detail).

The bending portion 6 has a distal end provided consecutively to a proximal end of the distal end portion 5. The bending portion 6 is a tubular member configured to be actively bendable in accordance with an operation input of a bending operation knob 9 which will be described later. The flexible tube portion 7 has a distal end provided consecutively to a proximal end of the bending portion 6. The flexible tube portion 7 has a proximal end provided consecutively to a distal end of the operation section 3. The flexible tube portion 7 is formed of a flexible tube having flexibility.

The operation section 3 is provided consecutively to a proximal end of the insertion section 2. The operation section 3 is configured to have a treatment instrument insertion opening 8, the bending operation knob 9, the forceps lever 10, a bending fixing lever 11, a plurality of operation members 12, and the like.

The treatment instrument insertion opening 8 is an opening part communicating with the treatment instrument insertion conduit (not shown) allowed to be inserted through and arranged in the insertion section 2. The treatment instrument insertion opening 8 is provided with a forceps plug 8a which is a forceps pipe sleeve.

The bending operation knob 9 is an operation member for performing a bending operation on the bending portion 6. The bending operation knob 9 is composed of two operation members for an up/down bending operation and a left/right bending operation. The two operation members of the bending operation knob 9 are disposed on an outer surface of the operation section 3 in an overlapping form. In this case, the two operation members of the bending operation knob 9 are disposed to be rotatable on a first rotation axis Ax which is an identical central axis.

The forceps lever 10 is an operation member for inputting an operational force for performing a raising operation of the forceps raising base (not shown) which is a movable member provided in the distal end portion 5. The forceps lever 10 is configured to be turnable within a predetermined range using the first rotation axis Ax identical to the central axis of the bending operation knob 9 as the rotation center. The forceps lever 10 is coupled to the forceps raising base through the driving mechanism 30 (not shown in Fig. 1; see Fig. 12 which will be described later) disposed inside the operation section 3 and the traction wire 31 (see Fig. 12) allowed to be inserted through the insertion section 2 (which will be described later in detail).

With this configuration, an operation input of the forceps lever 10 is transmitted to the forceps raising base. Thus, the forceps raising base may change the protruding direction of the treatment instrument (not shown) in the state allowed to be inserted through the treatment instrument insertion conduit (not shown) to be caused to protrude to the outside of the distal end portion 5.

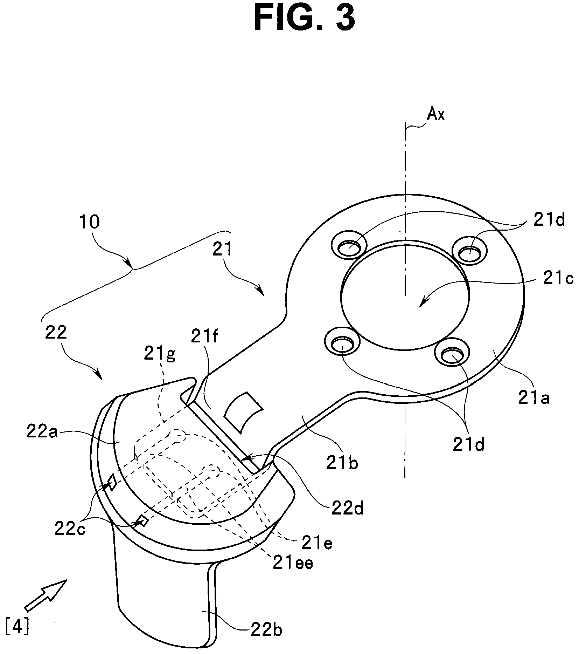

Note that the forceps lever 10 is configured to be turnable within a predetermined range using the first rotation axis Ax as the rotation center as described above. In this case, the forceps lever 10 turns within a predetermined range in directions of arrows R as shown in Fig. 2. Herein, the predetermined turning range is defined by the forceps lever 10 turning in the directions of the arrows R so that a portion (a side portion) of a projection 22b of the forceps lever 10 comes into contact with a portion (see reference numerals 3xd, 3xu in Fig. 2) of an exterior wall surface of the operation section 3. Note that a detailed configuration of the forceps lever 10 will be described later (see Fig. 3 and subsequent diagrams).

The bending fixing lever 11 is an operation member for acting on the bending operation knob 9 to fix a bending state of the bending portion 6 at a desired position. The bending fixing lever 11 is configured to be turnable within a predetermined range using the first rotation axis Ax as the rotation center. Note that a bending fixing mechanism for the bending portion 6 achieved by the bending fixing lever 11 is a portion not directly pertinent to the present invention. Therefore, a detailed configuration and illustration of the bending fixing lever 11 and the bending fixing mechanism will be omitted assuming that the bending fixing lever 11 and the bending fixing mechanism have a configuration similar to the configuration of a bending fixing lever and a bending fixing mechanism applied to a conventional endoscope.

The plurality of operation members 12 include, for example, operation switches for performing predetermined operations and the like as appropriate on image data acquired by the endoscope 1. Examples of the predetermined operations on image data include an operation of switching between and displaying a moving image and a still image, an operation of displaying an image under magnification, an image recording operation, image editing, and the like.

Examples of the plurality of operation members 12 include operation members that perform operations such as a gas/liquid feeding operation and a suction operation. Herein, the gas/liquid feeding operation is an operation that controls a motion of delivering a fluid from a fluid delivering part (not shown) provided in the distal end portion 5. The suction operation is an operation that controls the pressure in the treatment instrument insertion conduit (not shown) to be negative. By performing the suction operation, mucus adhering to the distal end portion 5, for example, can be suctioned and removed through an opening part (not shown) of the treatment instrument insertion conduit provided in the distal end portion 5.

The universal cable 4 is a composite cable extending from a side surface of the operation section 3. A scope connector 14 is disposed at a distal end of the universal cable 4. The scope connector 14 is a connector member connected to a video processor (not shown) which is an external device including a light source device. The scope connector 14 has a light guide connector 13 and the like in addition to an electric contact and the like.

A light guide bundle 15, various signal lines 16, and the like are allowed to be inserted and arranged between the scope connector 14 and the distal end portion 5 of the insertion section 2 through the universal cable 4 and the operation section 3. The light guide bundle 15 is a constituent member that transfers light flux emitted from the light source device included in the video processor not shown to the distal end portion 5 of the insertion section 2. The light flux transferred to the distal end portion 5 by the light guide bundle 15 is emitted from an illumination unit (not shown) provided in the distal end portion 5 as illumination light that illuminates an observation target.

Examples of the various signal lines 16 include an image pickup cable that transfers an image signal (image data) acquired by an image pickup unit (not shown) provided in the distal end portion 5 to the video processor (not shown), a control signal cable that transfers a control signal issued from the video processor to a constituent unit such as the image pickup unit, and the like. The foregoing is the schematic configuration of the endoscope 1.

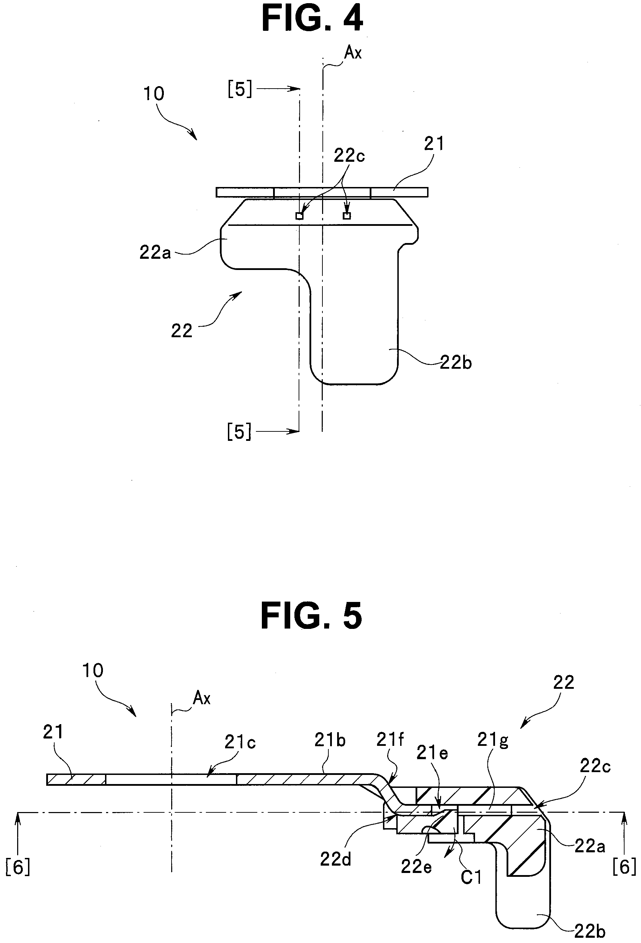

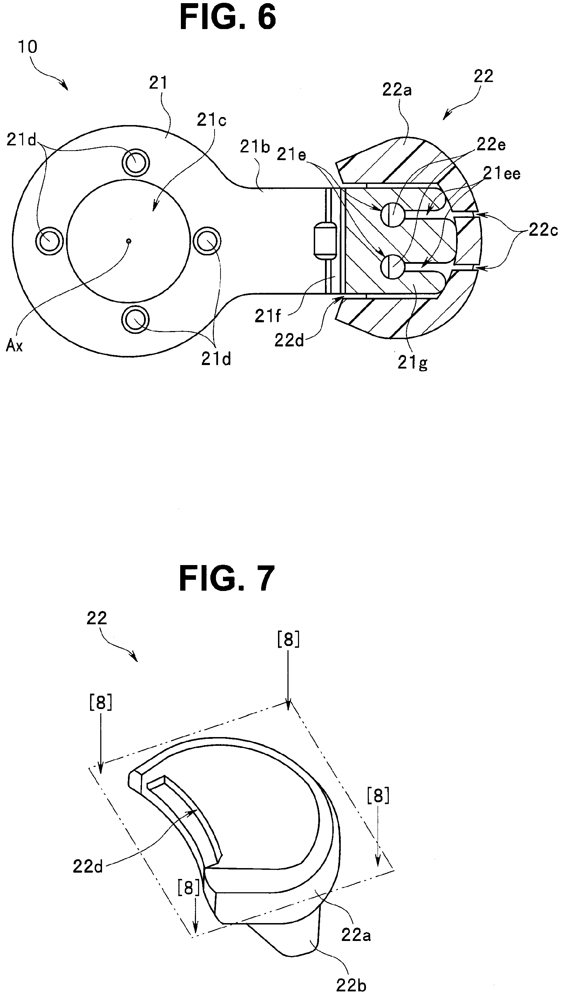

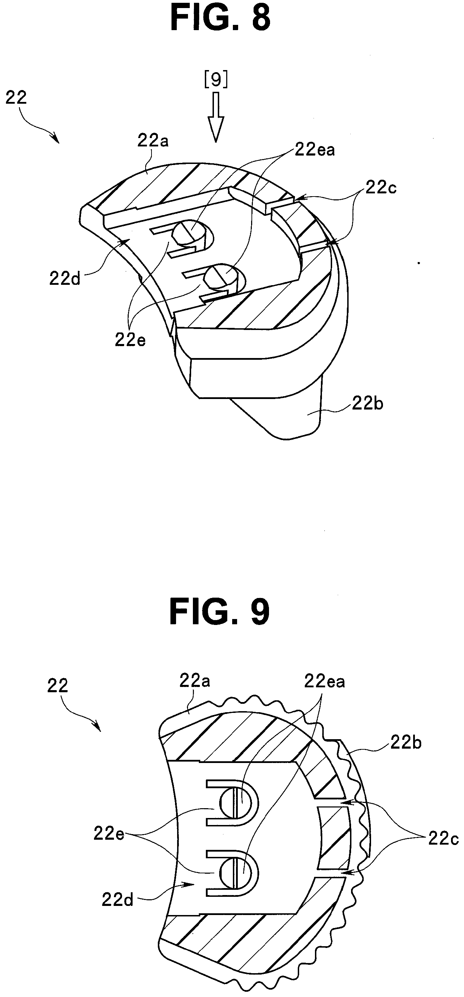

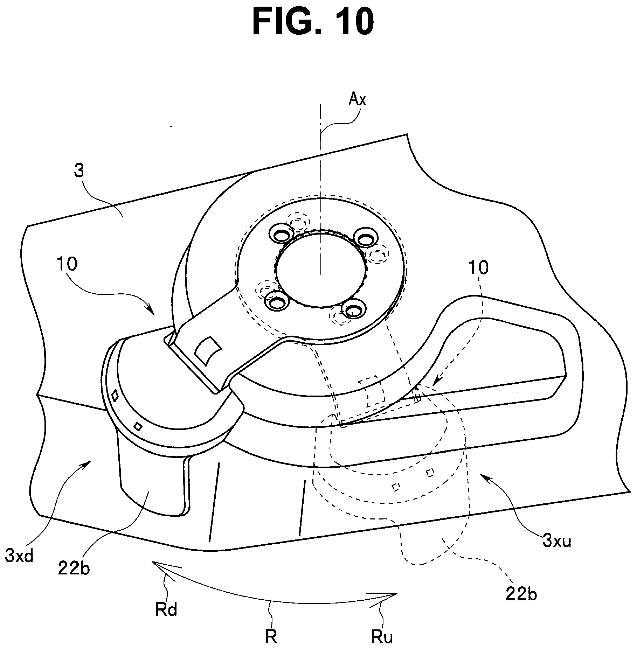

Next, a detailed configuration of the forceps lever 10 in the endoscope 1 of the first embodiment will be described below with reference to Fig. 3 to Fig. 10. Fig. 3 is an external perspective view showing only the forceps lever in the endoscope in Fig. 1 having been taken out. Fig. 4 is a plan view as seen in a direction indicated by an arrow sign [4] in Fig. 3. Fig. 5 is a cross-sectional view along a line [5]-[5] in Fig. 4. Fig. 6 is a cross-sectional view along a line [6]-[6] in Fig. 5. Fig. 7 is an external perspective view only showing a finger rest member in the forceps lever in Fig. 3 having been taken out. Fig. 8 is a cross-sectional view taken along a plane indicated by signs [8] in Fig. 7. Fig. 9 is a diagram showing the cross-section in Fig. 8 as seen in a direction indicated by an arrow sign [9]. Fig. 10 is a diagram showing a turning range of the forceps lever in Fig. 3 during normal use.

The forceps lever 10 in the endoscope 1 of the first embodiment is an operation member that upon receipt of an operational force inputted (operated) by a user, transmits the input to the driving mechanism 30 (which will be described later in detail; see Fig. 12 and the like) provided inside the operation section 3. The forceps lever 10 is provided to be turnable around the first rotation axis Ax which is a central axis of an internal fixing member (a frame shaft 36 which will be described later; see Fig. 12 to Fig. 14 and the like) of the operation section 3. The forceps lever 10 is configured by a lever member 21 and a finger rest member 22.

The lever member 21 is formed by subjecting a thin metal plate member, for example, to a bending process or the like. The lever member 21 is formed to have a main body portion 21a, an operation arm portion 21b, a shaft insertion hole 21c, a plurality of screw holes 21d, and to-be-locked holes 21e.

The main body portion 21a is an area formed into a substantially annular shape and fixed to a link main body 35 (see Fig. 12 and the like) which will be described later.

The operation arm portion 21b is an area formed into an arm shape protruding from an area of an outer periphery portion of the main body portion 21a toward a radially outer side. A stepped part 21f is provided through a bending process in the neighborhood of a distal end of the operation arm portion 21b. A cover attachment portion 21g having a plane parallel to planes of the main body portion 21a and the operation arm portion 21b is formed on the distal end side of the stepped part 21f. Note that herein, the stepped part 21f is not necessarily an essential component.

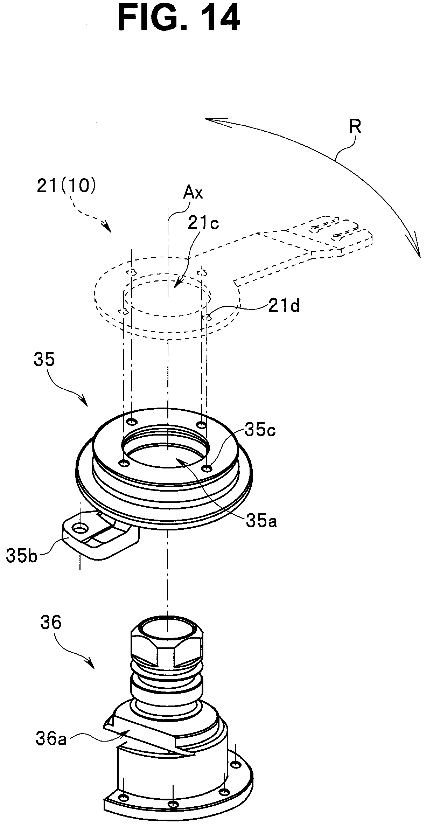

The shaft insertion hole 21c is a hole formed in a substantially central region of the main body portion 21a. The shaft insertion hole 21c is a hole that allows insertion and arrangement of the frame shaft 36 (see Fig. 14 and the like) which will be described later.

The plurality of screw holes 21d are small-diameter through-holes formed in an outer periphery region of the shaft insertion hole 21c of the main body portion 21a. The plurality of screw holes 21d are holes that allow insertion of fastening screws (not shown) for fixing the lever member 21 to the link main body 35 which will be described later.

The to-be-locked holes 21e are holes for locking lever lock portions 22e (which will be described later; see Fig. 5 and the like) when the finger rest member 22 is mounted on the lever member 21. When the lever lock portions 22e are locked in the to-be-locked holes 21e, the finger rest member 22 is maintained in the state mounted on the lever member 21. Note that the to-be-locked holes 21e have through-grooves 21ee formed to extend to a distal end edge of the cover attachment portion 21g in a direction along the cover attachment portion 21g.

The finger rest member 22 is a cover member for the user to rest his/her fingers or the like when operating the forceps lever 10. The finger rest member 22 is formed of a resin member, for example. The finger rest member 22 is configured to have a main body portion 22a, a projection 22b, jig insertion grooves 22c, a lever insertion opening 22d, the lever lock portions 22e (not shown in Fig. 3; see Fig. 5 and the like), and the like.

The main body portion 22a is composed of an enclosure having a substantially semi-circular plane and having an internal space. The main body portion 22a is formed to cover the cover attachment portion 21g when attached to the lever member 21. Herein, when the finger rest member 22 is in the mounted state mounted on the lever member 21, the substantially semi-circular plane of the main body portion 22a is disposed on a plane perpendicular to the first rotation axis Ax.

The projection 22b is an area formed into a thin plate shape protruding from the neighborhood of an outer periphery portion on the distal end side of the main body portion 22a in a second direction parallel to the direction along the first rotation axis Ax. Herein, the projection 22b is formed integrally with the main body portion 22a.

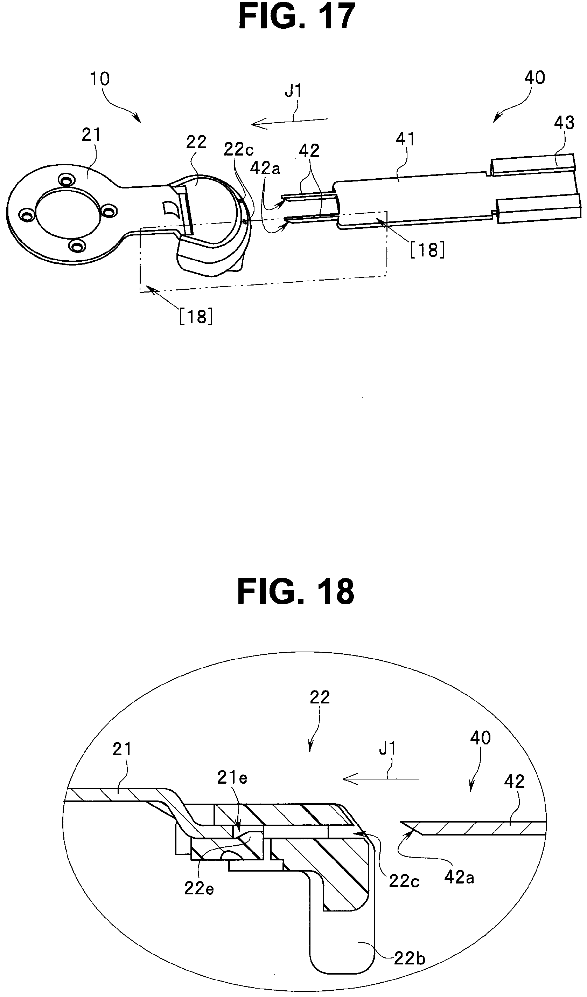

The jig insertion grooves 22c are areas formed for inserting portions of a predetermined jig (a removal jig 40 in Fig. 17 which will be described later and the like) to be used when detaching the finger rest member 22 mounted on the lever member 21. The jig insertion grooves 22c are grooves that allow insertion of the jig from openings formed in an outer surface of the main body portion 22a toward an internal space of the main body portion 22a in a direction parallel to the substantially semi-circular plane of the main body portion 22a. The internal space of the main body portion 22a communicates with the outside through the jig insertion grooves 22c.

The lever insertion opening 22d is an opening through which the cover attachment portion 21g is inserted when mounting the finger rest member 22 on the lever member 21. The lever insertion opening 22d is opened in the outer surface of the main body portion 22a and communicates with the internal space of the main body portion 22a.

The lever lock portions 22e are holes formed to be fitted into the to-be-locked holes 21e of the cover attachment portion 21g in the state in which the finger rest member 22 is mounted on the lever member 21 to maintain the mounted state of the finger rest member 22. Thus, in a normal state, the lever lock portions 22e are disposed to protrude from a floor surface of the internal space of the main body portion 22a toward the inside.

In further detail, the lever lock portions 22e are formed in a cantilever fashion as shown in Fig. 5, Fig. 8, Fig. 9, and the like. Herein, the lever lock portions 22e are formed to be swingable in a direction of an arrow C1 shown in Fig. 5. In the normal state, the lever lock portions 22e are arranged with free-end neighboring regions 22ea protruding toward the internal space of the main body portion 22a as shown in Fig. 8 and the like.

When the cover attachment portion 21g is inserted through the lever insertion opening 22d, the lever lock portions 22e may bow in the direction of the arrow C1 to pass the cover attachment portion 21g. When the cover attachment portion 21g advances to positions at which the free-end neighboring regions 22ea coincide with the to-be-locked holes 21e, the lever lock portions 22e return to the normal state and bowing is released. Then, the free-end neighboring regions 22ea are locked in the to-be-locked holes 21e. The finger rest member 22 is thus maintained in the state mounted on the lever member 21, and is brought into a state not being easily detached.

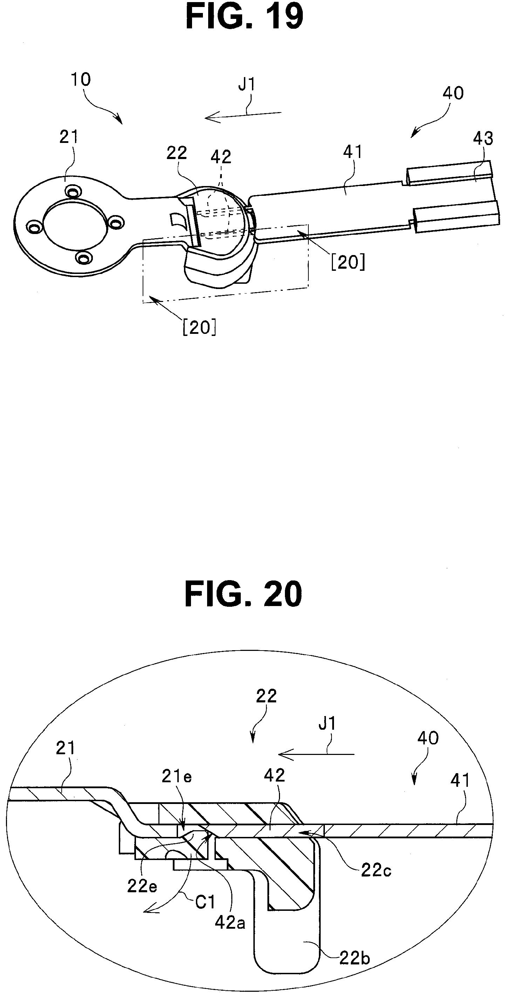

Although the details will be described later, when the removal jig 40 (see Fig. 17 and the like) is inserted through the jig insertion grooves 22c, the lever lock portions 22e similarly bow in the direction of the arrow C1 to release the locked state of the free-end neighboring regions 22ea and the to-be-locked holes 21e (which will be described later in detail; see Fig. 19 to Fig. 21 and the like).

The forceps lever 10 thus configured is arranged on an exterior surface of the operation section 3 as shown in Fig. 2, Fig. 10, and the like. In this case, the forceps lever 10 is disposed to be turnable within a predetermined range in the directions of the arrows R using the first rotation axis Ax as the rotation center.

In other words, the forceps lever 10 is held to be turnable around the first rotation axis Ax. When the forceps lever 10 receives an operation input made by the user, the forceps lever 10 turns in the directions along the arrows R in Fig. 10. As described above, when the forceps lever 10 turns, the forceps raising base (not shown) which is the movable member provided in the distal end portion 5 is moved in a predetermined direction (which will be described later in detail) by action of the driving mechanism 30 and the traction wire 31 which will be described later.

Note that the turning range of the forceps lever 10 in the directions of the arrows R is defined by the projection 22b of the finger rest member 22 of the forceps lever 10 coming into contact with a predetermined wall surface (3xd, 3xu) of the operation section 3. In this case, the wall surfaces (3xd, 3xu) of the operation section 3 serve as stoppers that define the turning range of the forceps lever 10.

Specifically, when the forceps lever 10 is located at a position indicated by a solid line in Fig. 10 and turns in the direction of an arrow Ru in Fig. 10 upon receipt of a predetermined operation input, a side portion of the projection 22b of the finger rest member 22 of the forceps lever 10 comes into contact with the first wall surface 3xu before long. When the forceps lever 10 thus reaches a position indicated by a dotted line in Fig. 10, the forceps lever 10 is restricted from turning. In other words, at this time, the first wall surface 3xu functions as the stopper that restricts the forceps lever 10 from turning.

Similarly, when the forceps lever 10 is located at the position indicated by the dotted line in Fig. 10 and turns in the direction of an arrow Rd in Fig. 10 upon receipt of a predetermined operation input, the other side portion of the projection 22b of the finger rest member 22 of the forceps lever 10 comes into contact with the first wall surface 3xd before long. When the forceps lever 10 thus reaches the position indicated by the solid line in Fig. 10, the forceps lever 10 is restricted from turning. In other words, at this time, the first wall surface 3xd functions as the stopper that restricts the forceps lever 10 from turning.

As described above, the operation section 3 provided on the proximal end side of the insertion section 2 in the endoscope 1 of the present embodiment is configured to have the forceps lever 10 and the stoppers (the first wall surfaces 3xd and 3xu constituting portions of the exterior wall surface).

Note that the position of the forceps lever 10 at which the projection 22b of the finger rest member 22 of the forceps lever 10 is in contact with the first wall surface (3xd, 3xu) shall be referred to as a first position. Herein, when in the state in which the forceps lever 10 is located at the first position and the projection 22b is in contact with the first wall surface 3xd, the forceps raising base which is the movable member is restricted from moving in an inverting direction. When in the state in which the forceps lever 10 is located at the first position and the projection 22b is in contact with the first wall surface 3xu, the forceps raising base which is the movable member is restricted from moving in a raising direction.

When the forceps lever 10 is arranged at the position indicated by the solid line in Fig. 10, the endoscope 1 of the present embodiment is in an unloaded state. Therefore, the state at this time will be called a normal state in the following description.

As described above, when the forceps lever 10 is located at the first position and the endoscope 1 is in the normal state, the first wall surface 3xd which is the stopper is in contact with the projection 22b of the finger rest member 22 of the forceps lever 10.

When the forceps lever 10 is located at the first position, that is, when the projection 22b of the finger rest member 22 of the forceps lever 10 is in contact with either of the first wall surfaces 3xd and 3xu which are the stoppers, the forceps raising base (not shown; which will be described later in detail) is arranged at a normal inverted position or a predetermined maximum raised position.

Herein, a schematic configuration of the forceps raising base which is the movable member will be described below with reference to Fig. 11. Fig. 11 is a perspective view showing a schematic configuration including the forceps raising base which is the movable member provided in the distal end portion of the insertion section in the endoscope of the first embodiment.

In the endoscope 1 of the present embodiment, a forceps raising base 54 is disposed together with a rotation axis member 54a inside the distal end portion 5 of the insertion section 2 as shown in Fig. 11. In this case, the forceps raising base 54 is a movable member configured to be movable in a predetermined first direction. Herein, the first direction is a circumferential direction with a second rotation axis Ax2 of the rotation axis member 54a shown by a dotted line in Fig. 11 serving as a central axis. The forceps raising base 54 is configured to be turnable around the second rotation axis Ax2 within a predetermined turning range (see an arrow sign T in Fig. 11).

The distal end portion 5 is configured by a main body 50 and a distal end cover not shown. An illumination unit 51, an image pickup unit 52, a gas/liquid feeding nozzle 53, the forceps raising base 54, a portion of the traction wire 31, and the like are disposed on the main body 50.

The illumination unit 51 is configured to include an illumination optical member or the like provided to receive illumination light transmitted from the light source device (not shown) via the light guide bundle 15 and illuminate a predetermined range with the illumination light in a predetermined direction outside the distal end portion 5 and composed of an optical element and the like. Herein, an optical element disposed to be exposed at the outer surface of the distal end portion 5 on the side closest to the distal end of the illumination optical member shall be particularly referred to as an illumination window. Note that the illumination unit 51 itself has a configuration similar to the configuration of an illumination unit mounted on a conventional endoscope. Therefore, detailed description of the illumination unit 51 will be omitted.

The image pickup unit 52 is a constituent member configured by an image pickup optical system, an image pickup device, an image pickup device driving circuit, and the like. The image pickup optical system is composed of an optical lens that forms an optical image of an observation target, and the like. Note that an optical element disposed closest to the distal end side of the image pickup optical system in a manner exposed at the outer surface of the distal end portion 5 shall be particularly referred to as an observation window.

The image pickup device is an electronic component that receives an optical image formed by the image pickup optical system and performs photoelectric conversion. The image pickup device is driven by the image pickup device driving circuit. The image pickup device driving circuit receives an instruction signal from the video processor (not shown), and drives the image pickup device. Image data obtained by the image pickup device through photoelectric conversion is transmitted to the video processor through the image pickup device driving circuit. The video processor receives the received image data, and performs predetermined image data processing. Note that the image pickup unit 52 itself has a configuration similar to the configuration of an image pickup unit mounted on a conventional endoscope. Therefore, detailed description of the image pickup unit 52 will be omitted.

The gas/liquid feeding nozzle 53 is a constituent member that discharges a liquid or gas when performing liquid feeding or gas feeding from the distal end portion 5 toward the outside. For example, when a predetermined operation member among the plurality of operation members 12 provided in the operation section 3 is operated to perform a predetermined liquid feeding operation, a liquid for cleaning an outer surface of the observation window of the image pickup unit 52, the illumination window of the illumination unit 51, or the like is discharged through the gas/liquid feeding nozzle 53. Note that a gas/liquid feeding mechanism including the gas/liquid feeding nozzle 53 has a configuration similar to the configuration of a gas/liquid feeding mechanism mounted on a conventional endoscope. Therefore, detailed description of the gas/liquid feeding mechanism will be omitted.

A distal end opening 50a is formed in the main body 50. The treatment instrument insertion conduit (not shown) allowed to be inserted and arranged in the insertion section 2 is connected to the distal end opening 50a. Thus, the treatment instrument (not shown) inserted through the treatment instrument insertion opening 8 is allowed to be inserted through the treatment instrument insertion conduit, and the distal end portion of the treatment instrument protrudes outward from the distal end opening 50a. When a predetermined raising motion for the forceps raising base 54 is performed at this time, the protruding direction of the distal end portion of the treatment instrument can be changed.

The forceps raising base 54 is disposed on the distal end portion 5 to be rotatable around the second rotation axis Ax2 shown in Fig. 11. The second rotation axis Ax2 is a rotary axis substantially perpendicular to an insertion axis F (see Fig. 11) of the distal end portion 5. Herein, the arrows T in Fig. 11 indicate turning directions of the forceps raising base 54 around the second rotation axis Ax2. Of the arrows, an arrow sign T1 in Fig. 11 indicates the direction in which the forceps raising base 54 is raised (hereinafter, referred to as the raising direction). An arrow sign T2 in Fig. 11 indicates the direction in which the forceps raising base 54 is inverted (hereinafter, referred to as the inverting direction). In this case, rotation of the forceps raising base 54 is restricted to fall within a predetermined range by restricting the forceps lever 10 from turning as described above.

A distal end of the traction wire 31 extending from a distal end opening 50b provided in the main body 50 of the distal end portion 5 is coupled to a wire coupling portion 54b of the forceps raising base 54. The traction wire 31 is allowed to be inserted and arranged in the insertion section 2 and the operation section 3, and has a proximal end coupled to the driving mechanism 30 in the operation section 3.

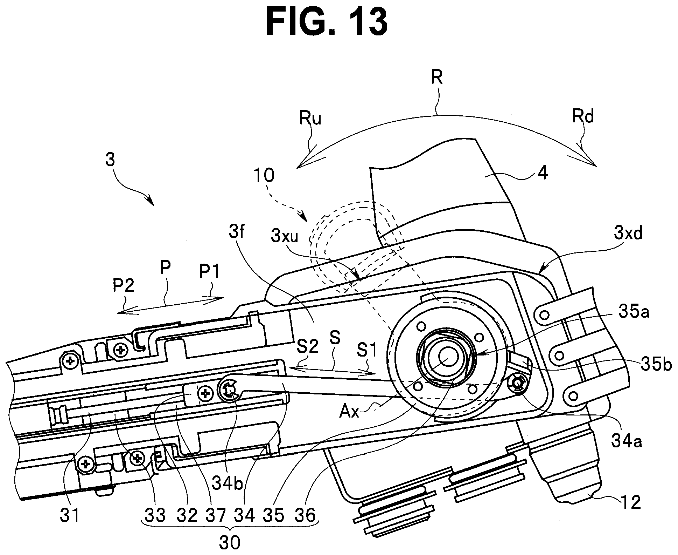

Subsequently, a configuration of the driving mechanism 30 of the forceps raising base 54 will be described below with reference to Fig. 12 to Fig. 16. Fig. 12 and Fig. 13 are plan views showing a schematic configuration of the driving mechanism for the forceps lever in the internal configuration of the operation section in the endoscope of the first embodiment. Fig. 12 is a diagram showing a condition of the forceps lever and the driving mechanism for the forceps lever when the forceps raising base is in the maximum inverted state. Fig. 13 is a diagram showing a condition of the forceps lever and the driving mechanism for the forceps lever when the forceps raising base is in the maximum raised state. Note that Fig. 12 and Fig. 13 merely show the outer shape of the forceps lever 10 by a dotted line in order to avoid complicating the drawings.

Fig. 14 is an essential part exploded perspective view showing some constituent members (the frame shaft and the link main body) in the driving mechanism for the forceps lever in Fig. 12 having been taken out. Note that in Fig. 14, the forceps lever 10 is indicated by a dotted line. At the same time, in the drawing, only the lever member 21 is shown in the forceps lever 10, and illustration of the finger rest member 22 is omitted.

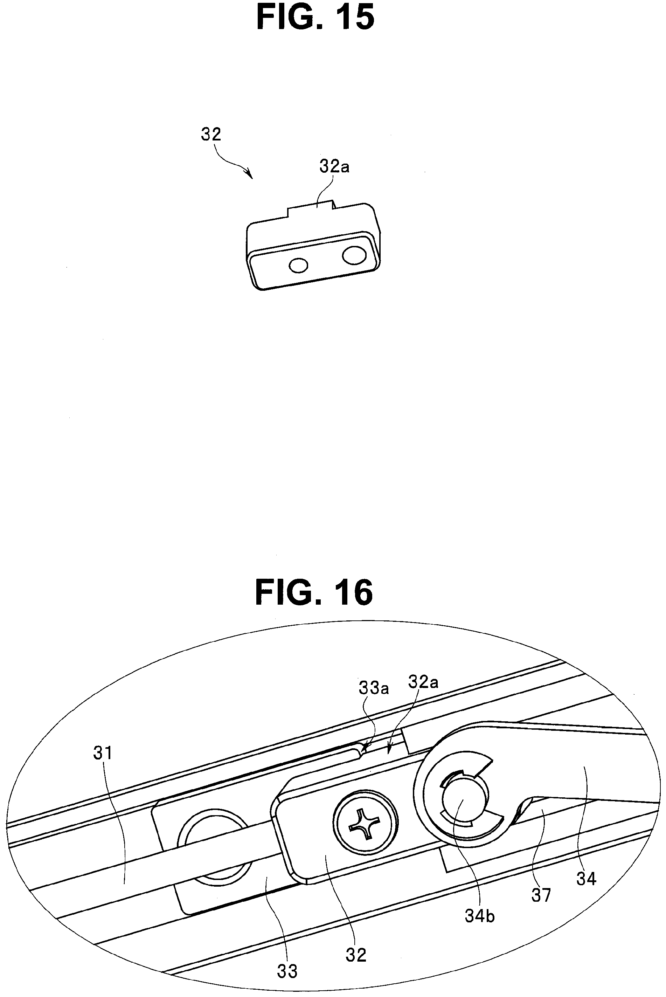

Fig. 15 is an essential part magnified perspective view showing only a partial constituent member (the coupling member) of the driving mechanism for the forceps lever in Fig. 12 having been taken out. Fig. 16 is an essential part magnified view showing under magnification a partial region (a neighboring region in a state in which the coupling member is in contact with the lock plate) of the driving mechanism for the forceps lever in Fig. 12.

The driving mechanism 30 in the endoscope 1 of the present embodiment is a link mechanism that receives an operation input of the forceps lever 10 and converts the operation input into an amount of force that moves the traction wire 31 forward and backward in the long axis direction of the insertion section 2. The traction wire 31 moved forward and backward through the driving mechanism 30 turns the forceps raising base 54 around the second rotation axis Ax2.

The driving mechanism 30 is a constituent unit attached and fixed to a frame 3f which is an internal fixing member of the operation section 3 as shown in Fig. 12 and the like. The driving mechanism 30 is configured by the frame shaft 36, the link main body 35, a rod body 34, a lock plate 33, a coupling member 32, a coupling member guide 37, and the like.

The frame shaft 36 is a shaft-like member in the form of being substantially upright with respect to a plane of the frame 3f of the operation section 3. The frame shaft 36 is fixed onto the plane of the frame 3f by screwing, for example. Herein, the frame shaft 36 is disposed with the central axis matched with the first rotation axis Ax serving as the rotation center of the forceps lever 10.

As shown in Fig. 14, the frame shaft 36 has a notch 36a formed partially on an outer circumferential surface. Herein, the frame shaft 36 is formed such that a plane including an area in which the notch 36a is arranged, which is a plane substantially perpendicular to the second direction parallel to the direction along the first rotation axis Ax, has a substantially D-shaped cross-section.

The link main body 35 is a member having a substantially annular shape as a whole and disposed to be turnable around the frame shaft 36 (in the directions of the arrows R in Fig. 14) as shown in Fig. 14. Thus, the link main body 35 is formed to have a through-hole 35a to be fitted over the frame shaft 36 substantially in a central region.

In the link main body 35, a link portion 35b is formed partially on an outer periphery portion to protrude outward. A first end 34a on a proximal end side of the rod body 34 which will be described later is coupled to the link portion 35b as shown in Fig. 12 and the like.

The link main body 35 further has a plurality of screw holes 35c formed on an annular region. The screw holes 35c are areas in which a plurality of fastening screws (not shown) for fixing the lever member 21 of the forceps lever 10 are fastened, respectively.

The rod body 34 is a link member that converts a rotation motion of the link main body 35 into a linear motion. As described above, the link portion 35b is coupled to the first end 34a on the proximal end side of the rod body 34. The coupling member 32 which will be described later is coupled to a second end 34b on a distal end side of the rod body 34 as shown in Fig. 12 and the like.

The coupling member 32 is a constituent member that couples the driving mechanism 30 and the traction wire 31. The second end 34b on the distal end side of the rod body 34 is coupled to the coupling member 32 as described above, and a proximal end of the traction wire 31 is coupled to the coupling member 32. The coupling member 32 is disposed to be movable in predetermined directions (directions along the long axis of the insertion section 2, which are directions of arrows P in Fig. 12 and Fig. 13) within the coupling member guide 37 which will be described later.

The coupling member 32 has a protruding portion 32a formed on a bottom surface portion as shown in Fig. 15 and Fig. 16. The protruding portion 32a has a function of restricting the coupling member 32 at a predetermined position from moving in the direction of an arrow P2 which is one of the directions of the arrows P. In other words, when the coupling member 32 moves in the direction of the arrow P2, an end surface of the protruding portion 32a closer to the distal end comes into contact with a proximal end side end surface 33a of the lock plate 33 which will be described later, thus restricting the movement (see Fig. 16).

The coupling member guide 37 is a constituent member that guides a movement of the coupling member 32 in predetermined directions (the directions of the arrows P). The coupling member guide 37 is fixed onto the plane of the frame 3f of the operation section 3. In this case, the coupling member guide 37 is formed to extend parallel to the plane of the frame 3f and in a direction in the long axis direction of the operation section 3. The coupling member 32 is housed to be movable in the coupling member guide 37 as described above. Along with this, the lock plate 33 is fixed in the coupling member guide 37.

The lock plate 33 is a constituent member for restricting the coupling member 32 that moves in the coupling member guide 37 from moving in a predetermined direction at a predetermined position. The lock plate 33 is fixed to a predetermined area within the coupling member guide 37.

With such a configuration, when the link main body 35 receives an operation input of the forceps lever 10 and turns around the first rotation axis Ax (the directions of the arrows R in Fig. 12 and the like), the rod body 34 moves in directions of arrows S in Fig. 12 and the like. Then, at this time, the coupling member 32 slides inside the coupling member guide 37 to move forward and backward in the long axis direction of the insertion section 2 (the directions of the arrows P in Fig. 12 and the like).

Herein, for example, the driving mechanism 30 in a state in which the forceps lever 10 is detached acts as described below. For example, assume that the link main body 35 turns in the direction of the arrow Rd when in the state shown in Fig. 13. Then, the rod body 34 moves in the direction of an arrow S2 in Fig. 13, and the coupling member 32 moves in the direction of the arrow P2 in Fig. 13. Then, the driving mechanism 30 is brought into the state shown in Fig. 12 before long.

At this time, a distal end surface of the protruding portion 32a (see Fig. 15 and Fig. 16) of the coupling member 32 comes into contact with the proximal end side end surface 33a of the lock plate 33 as shown in Fig. 16. This restricts the coupling member 32 from moving in the same direction (the direction of the arrow P2). At this time, the coupling member 32 is arranged at a position closest to the distal end in a movable range in the coupling member guide 37.

In this case, the lock plate 33 restricts the coupling member 32 (and the traction wire 31) from moving to a position closer to the distal end in the long axis direction (the directions of the arrows P) of the insertion section 2 (moving in the direction of the arrow P2). In other words, the lock plate 33 serves to define one end of the entire moving range of the coupling member 32 (and the traction wire 31) moved by the driving mechanism 30 in the long axis direction.

Assume that when in the state shown in Fig. 12, for example, the link main body 35 turns in the direction of the arrow Ru. Then, the rod body 34 moves in the direction of an arrow S1 in Fig. 12, and the coupling member 32 moves in the direction of an arrow P1 in Fig. 12. Then, the driving mechanism 30 is brought into the state shown in Fig. 13 before long.

At this time, in the driving mechanism 30, a middle portion of the rod body 34 comes into contact with an end surface of the notch 36a of the frame shaft 36. This restricts the rod body 34 from moving in the direction of the arrow S1. At the same time, the coupling member 32 is restricted from moving in the direction of the arrow P1. This restricts the link main body 35 from turning in the direction of the arrow Ru. At this time, the coupling member 32 is arranged at a position closest to the proximal end in the movable range in the coupling member guide 37. In this case, the notch 36a serves to define the other end of the entire moving range of the coupling member 32 (the traction wire 31) moved by the driving mechanism 30 in the long axis direction.

The driving mechanism 30 in a case where the forceps lever 10 is mounted on the link main body 35 of the driving mechanism 30 acts as described below. In other words, assume that when in the state shown in Fig. 13, the forceps lever 10 turns in the direction of the arrow Rd and the link main body 35 turns in the same direction. Then, the rod body 34 moves in the direction of the arrow S2 in Fig. 13, and the coupling member 32 moves in the direction of the arrow P2 in Fig. 13. The projection 22b of the finger rest member 22 of the forceps lever 10 comes into contact with the first wall surface 3xd (stopper) before long. This restricts the forceps lever 10 from turning in the direction of the arrow Rd. Therefore, the coupling member 32 is restricted from moving in the direction of the arrow P2.

In this state, the protruding portion 32a of the coupling member 32 is in a state before coming into contact with the proximal end side end surface 33a of the lock plate 33. In other words, a predetermined gap is present between a distal end surface of the protruding portion 32a and the proximal end side end surface 33a. This means that the coupling member 32 is in a state not having reached the one end in the entire moving range in the long axis direction by the driving mechanism 30.

Assume that when in the state shown in Fig. 12, the forceps lever 10 turns in the direction of the arrow Ru, and the link main body 35 turns in the same direction. Then, the rod body 34 moves in the direction of the arrow S1 in Fig. 12, and the coupling member 32 moves in the direction of the arrow P1 in Fig. 12. The projection 22b of the finger rest member 22 of the forceps lever 10 comes into contact with the first wall surface 3xu (stopper) before long. This restricts the forceps lever 10 from turning in the direction of the arrow Ru. Therefore, the coupling member 32 is restricted from moving in the direction of the arrow P1.

In this state, the rod body 34 is in a state before coming into contact with the end surface of the notch 36a of the frame shaft 36. In other words, a predetermined gap is present between the rod body 34 and the end surface of the notch 36a of the frame shaft 36. This means that the link main body 35 is in a state not having reached the other end in the turning range around the first rotation axis Ax by the driving mechanism 30. In other words, at this time, the coupling member 32 is in a state not having reached the other end in the entire moving range in the long axis direction by the driving mechanism 30.

Herein, in the endoscope 1 of the present embodiment, a state of the forceps lever 10 when the forceps lever 10 is attached to the driving mechanism 30 shall be referred to as a first state. A state in which the forceps lever 10 is detached from the driving mechanism 30 shall be referred to as a second state.

In the first state, when the projection 22b of the finger rest member 22 of the forceps lever 10 comes into contact with the first wall surface (3xu, 3xd) which is the stopper, the forceps lever 10 (and the link main body 35) is restricted from turning, so that the moving range of the traction wire 31 is restricted. In the second state, the moving range of the traction wire 31 is restricted only based on a structure essentially provided for the driving mechanism 30. In other words, in the second state, the link main body 35 is restricted from turning by contact between the protruding portion 32a of the coupling member 32 and the proximal end side end surface 33a of the lock plate 33 and contact between the rod body 34 and the notch 36a of the frame shaft 36, so that the moving range of the traction wire 31 is restricted.

However, in a state in which the endoscope 1 is assembled, it is considered difficult to easily produce the second state of the forceps lever 10 (that is, the state in which the forceps lever 10 is not mounted) because work such as disassembling the operation section 3 would follow.

Hence, in the endoscope 1 of the present embodiment, by configuring the forceps lever 10 such that the finger rest member 22 is removable from the lever member 21, the second state is produced easily. In other words, the second state of the forceps lever 10 can be achieved by detaching the finger rest member 22 from the lever member 21 in the forceps lever 10.

Herein, the configuration and action of making the finger rest member 22 of the forceps lever 10 removable from the lever member 21 in the endoscope 1 of the present embodiment will be described below in detail with reference to Fig. 17 to Fig. 26.

Fig. 17 is a schematic perspective view showing a configuration of the forceps lever and a finger-rest-member removal jig in the endoscope of the present embodiment. Fig. 18 is a partial cross-sectional view along a plane indicated by signs [18] in Fig. 17. Fig. 19 to Fig. 23 are diagrams showing an action when detaching the finger rest member from the forceps lever in the endoscope of the present embodiment. Of these diagrams, Fig. 19 is a schematic perspective view showing a course when detaching the finger rest member from the forceps lever, showing a state in which a jig is inserted in the jig insertion grooves of the finger rest member.

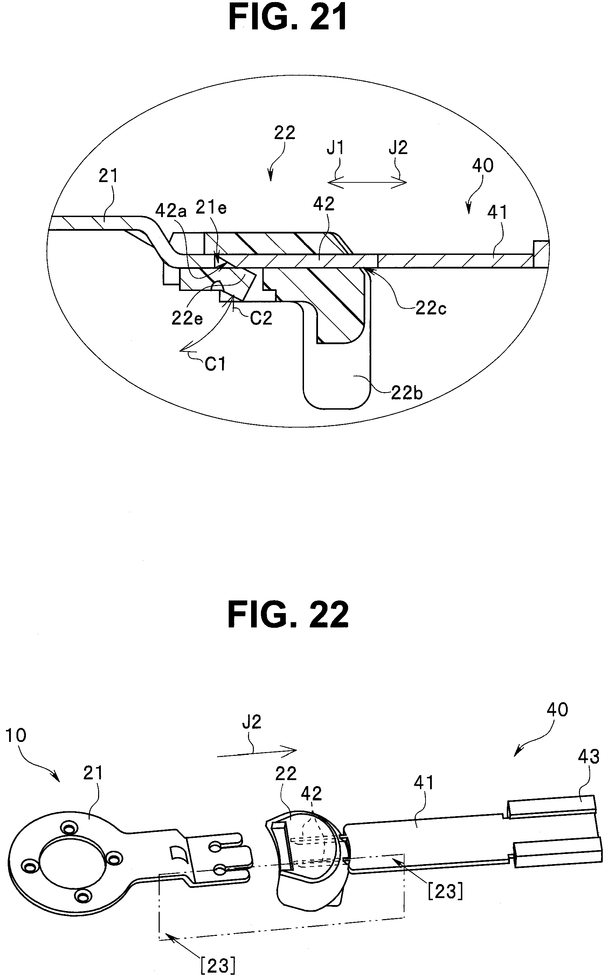

Fig. 20 and Fig. 21 are partial cross-sectional views along a plane indicated by signs [20] in Fig. 19. Of these diagrams, Fig. 20 shows a state in which the lever lock portion is locked in the to-be-locked hole. Fig. 21 shows a state in which a locked state of the lever lock portion in the to-be-locked hole is released.

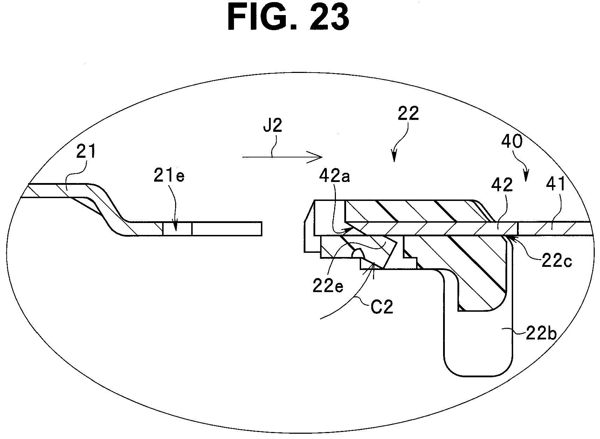

Fig. 22 is a schematic perspective view showing a course when detaching the finger rest member from the forceps lever, showing a state in which the finger rest member is detached by the jig after the state in Fig. 19. Fig. 23 is a partial cross-sectional view along a plane indicated by signs [23] in Fig. 22.

The detailed configuration of the forceps lever 10 in the endoscope 1 of the present embodiment has been described above with reference to Fig. 2 to Fig. 9.

A configuration of the removal jig 40 which is the finger-rest-member removal jig will be briefly described below. The removal jig 40 is a dedicated jig to be used when removing the finger rest member 22 of the forceps lever 10. The removal jig 40 includes a jig main body portion 41, needle portions 42, and a mounting portion 43 as shown in Fig. 17 and the like.

The jig main body portion 41 is formed of a substantially rectangular flat plate. The plurality of needle portions 42 are disposed on one end of the jig main body portion 41. The mounting portion 43 is formed on the other end of the jig main body portion 41.

The needle portions 42 have an elongated rod shape formed to be provided in a manner protruding from the one end of the jig main body portion 41 to the outside of the jig main body portion 41 in the long axis direction. Note that the present embodiment shows an example in which a plurality of (two) needle portions 42 are provided.

The needle portions 42 are inserted into the inner side of the jig insertion grooves 22c of the finger rest member 22 to act on the lever lock portions 22e. Thus, the needle portions 42 are formed to have a shape and thickness that the needle portions 42 may be inserted into the inner side of the jig insertion grooves 22c of the finger rest member 22. The needle portions 42 are also formed to have such a length that distal ends may reach the lever lock portions 22e when the needle portions 42 are inserted through openings of the jig insertion grooves 22c into the inner side of the grooves 22c.

The needle portions 42 are also formed to have distal end tilted surfaces 42a (see Fig. 18) in a distal end region. In this case, the distal end tilted surfaces 42a are formed to face downward in a state in which the removal jig 40 is in a posture in use (the state shown in Fig. 17). The distal end tilted surfaces 42a thus act on the lever lock portions 22e when the needle portions 42 are inserted into the inner side of the jig insertion grooves 22c to cause the lever lock portions 22e to bow in a direction to be separated from the to-be-locked holes 21e.

The mounting portion 43 has a structure for attaching the removal jig 40 to the cover attachment portion 21g of the lever member 21 after the finger rest member 22 is detached from the forceps lever 10.

Thus, the mounting portion 43 is formed to be capable of covering the outer circumference of the cover attachment portion 21g of the lever member 21. Specifically, for example, the mounting portion 43 is formed to include groove portions having a channel shaped cross-section along both side surfaces of the jig main body portion 41 in the long axis direction. The mounting portion 43 is formed on the other end opposite to the one end of the jig main body portion 41 on which the needle portions 42 are provided.

The removal jig 40 thus configured has a function of detaching the finger rest member 22 from the forceps lever 10. At the same time, the removal jig 40 has a function of being mounted on the cover attachment portion 21g after the finger rest member 22 is detached through the mounting portion 43 to elongate the operation arm portion 21b of the lever member 21.

An action when detaching the finger rest member 22 which is the cover member from the lever member 21 of the forceps lever 10 using the removal jig 40 will be briefly described below.

In a situation in which the finger rest member 22 is detached from the lever member 21 of the forceps lever 10, a situation is usually assumed in which the forceps lever 10 is assembled in a predetermined area of the operation section 3 of the endoscope 1. However, in Fig. 17 to Fig. 23, illustration of components surrounding the forceps lever 10 is omitted in order to avoid complicating the drawings, and only the forceps lever 10 as a target is illustrated alone.

First, as shown in Fig. 17 and Fig. 18, the removal jig 40 is arranged at a predetermined position on the forceps lever 10. Herein, the predetermined position of the removal jig 40 is a position at which distal ends of the needle portions 42 of the removal jig 40 are opposed to the openings of the jig insertion grooves 22c of the finger rest member 22. Note that at this time, the removal jig 40 has a posture in which the distal end tilted surfaces 42a of the needle portions 42 are arranged to face downward.

From the state, the removal jig 40 is advanced in a direction of an arrow J1 in Fig. 17 and Fig. 18. The two needle portions 42 are then inserted into the two jig insertion grooves 22c. The state shown in Fig. 19 and Fig. 20 is thus brought about.

From the state, the removal jig 40 is advanced as it is in the direction of the arrow J1. Then, the distal end tilted surfaces 42a of the needle portions 42 come into contact with outer edge ends of the lever lock portions 22e before long. Before the state is brought about, the lever lock portions 22e of the finger rest member 22 are in a state fitted within the to-be-locked holes 21e of the lever member 21.

From the state, the removal jig 40 is advanced further in the direction of the arrow J1. Then, the distal end tilted surfaces 42a act on the outer edge ends of the lever lock portions 22e before long to cause the lever lock portions 22e to bow in the direction of the arrow C1 in Fig. 20. At this time, when the distal end tilted surfaces 42a of the needle portions 42 press the lever lock portions 22e in the direction of the arrow J1, the lever lock portions 22e move along the distal end tilted surfaces 42a to smoothly bow in the direction of the arrow C1. Then, the lever lock portions 22e transition to a state shown in Fig. 21.

In the state shown in Fig. 21, fitting of the lever lock portions 22e within the to-be-locked holes 21e is released. When the needle portions 42 are advanced as they are in the direction of the arrow J1, the needle portions 42 move to a position at which the to-be-locked holes 21e are covered while maintaining the state in which the lever lock portions 22e are caused to bow. At this time, in the lever lock portions 22e, an amount of force in a direction of an arrow C2 works because of elastic resilience of the lever lock portions 22e. The amount of force of the lever lock portions 22e in the direction of the arrow C2 is an amount of force that presses the lever lock portions 22e against the needle portions 42.

Therefore, when the removal jig 40 is moved in a direction of an arrow J2 in Fig. 21 in the state shown in Fig. 21, the finger rest member 22 is separated and pulled out from the cover attachment portion 21g together with the removal jig 40. Thus, the finger rest member 22 is detached from the lever member 21 as shown in Fig. 22 and Fig. 23. At this time, the finger rest member 22 is in the state attached to the removal jig 40. However, at this time, the finger rest member 22 is in a state easily detachable from the removal jig 40 by being pulled out by the user.

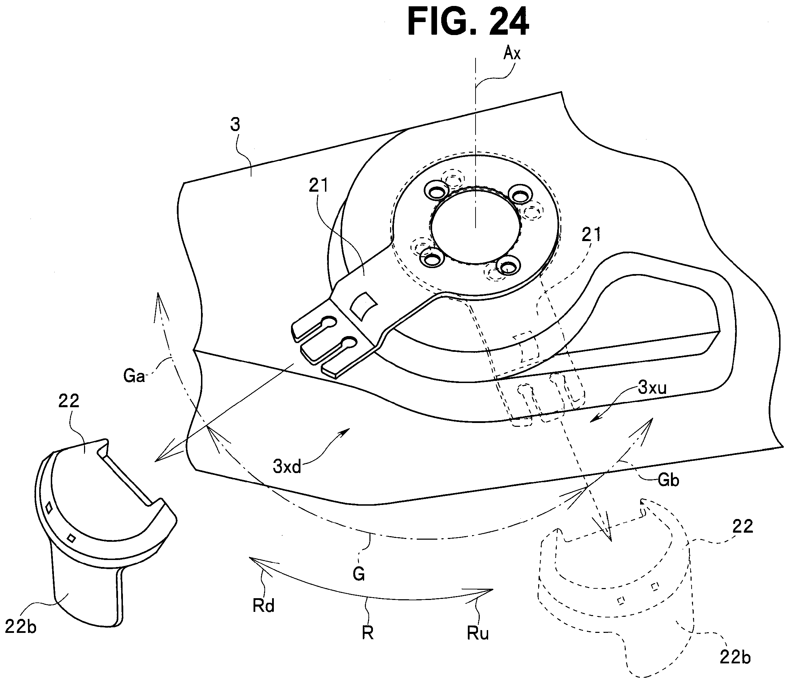

When the finger rest member 22 is detached from the forceps lever 10 in this manner, the lever member 21 of the forceps lever 10 is in a state left at a predetermined position of the operation section 3 as shown in Fig. 24. Fig. 24 shows a schematic configuration of the operation section after the finger rest member is detached from the forceps lever using the removal jig through the above-described detaching action.

Next, an action of the forceps lever (the lever member) in the state in which the finger rest member is detached will be described with reference to Fig. 24. In Fig. 24, a range indicated by arrows G indicated by a dashed-dotted line indicates the movable range of the lever member 21 when the forceps lever 10 is in the first state (the state in which the finger rest member 22 is mounted). A range indicated by arrows Ga and Gb indicated by the dashed-dotted line in Fig. 24 indicates a movable range of the lever member 21 added by bringing the forceps lever 10 into the second state (the state in which the finger rest member 22 is detached).

In the endoscope 1 of the present embodiment, by detaching the finger rest member 22 from the forceps lever 10 to bring about the second state, the movable range of the forceps lever 10 (the lever member 21) can be easily enlarged.

This will be described below using a specific exemplification. For example, in the first state in which the finger rest member 22 is mounted on the forceps lever 10 (see Fig. 10), the forceps lever 10 turns within the range indicated by the arrows G in Fig. 24.

In other words, in the first state, when the forceps lever 10 is located at the first position, the projection 22b comes into contact with the first wall surface (3xu, 3xd) which is the stopper, so that the turning range of the forceps lever 10 is restricted as described above.

In the second state (see Fig. 24) where the finger rest member 22 is detached from the forceps lever 10, for example, the forceps lever 10 turns within the range including ranges of the arrows Ga and Gb in addition to the range indicated by the arrows G in Fig. 24.

In this case, the range indicated by the arrow Ga is a range restricted by contact of the protruding portion 32a of the coupling member 32 and the proximal end side end surface 33a of the lock plate 33 in the driving mechanism 30 as described with reference to Fig. 12. The range indicated by the arrow Gb is a range restricted by contact of the rod body 34 and the notch 36a of the frame shaft 36.

In other words, since the finger rest member 22 is detached in the second state as described above, the projection 22b is in a state not coming into contact with the first wall surface (3xu, 3xd) which is the stopper when the lever member 21 of the forceps lever 10 is located at a position equivalent to the first position. Therefore, the lever member 21 of the forceps lever 10 can be turned further.

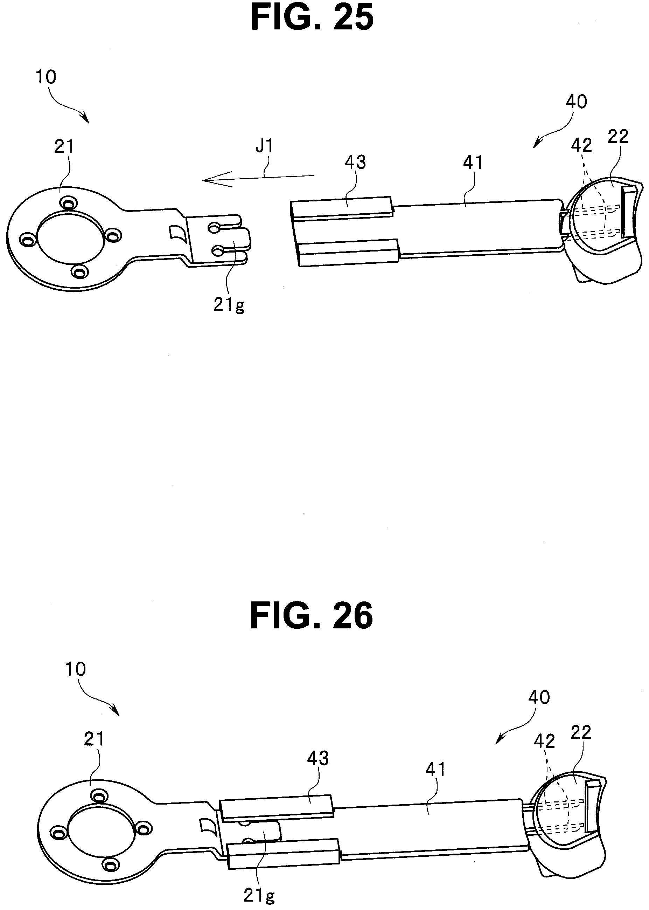

Note that herein, Fig. 25 and Fig. 26 show an action when attaching the mounting portion 43 of the removal jig 40 to the cover attachment portion 21g of the lever member 21 with the finger rest member 22 detached from the forceps lever 10.

When the forceps lever 10 is brought into the second state (the state in which the finger rest member 22 is detached) as shown in Fig. 24, the mounting portion 43 of the removal jig 40 is moved in the direction of the arrow J1 as shown in Fig. 25 and attached to the cover attachment portion 21g of the lever member 21, as shown in Fig. 25 and Fig. 26. Then, when a state in Fig. 26 is brought about, the operation arm portion 21b of the lever member 21 can be elongated by the length of the removal jig 40. Thus, the forceps lever 10 (the lever member 21) after the finger rest member 22 is detached can be turned without interfering with other constituent members on the operation section 3. This can improve the lever member 21 in operability even if the finger rest member 22 is detached.

As described above, the above-described first embodiment achieves the configuration in which the finger rest member 22 can be easily detached using the removal jig 40 from the forceps lever 10 for operating the forceps raising base which is the movable member provided in the distal end portion 5 among the operation members provided in the operation section 3 of the endoscope 1.

With this configuration, in the present embodiment, the forceps lever 10 can be easily deformed between the first state in which the finger rest member 22 is mounted and the second state in which the finger rest member 22 is detached.

By bringing the forceps lever 10 into the first state during the normal use of the endoscope 1, an appropriate movable range corresponding to the normal use can be set for the forceps lever 10. In the case of performing work such as repairing the endoscope 1, a wider movable range can be easily ensured for the forceps lever 10 (the lever member 21) without performing complicated work such as disassembling the operation section 3 by bringing the forceps lever 10 into the second state.

When the forceps lever 10 is brought into the second state, the forceps lever 10 (the lever member 21) in the state in which the finger rest member 22 is detached can be easily improved in operability by attaching the removal jig 40 to the lever member 21. Therefore, more efficient workability can be ensured.

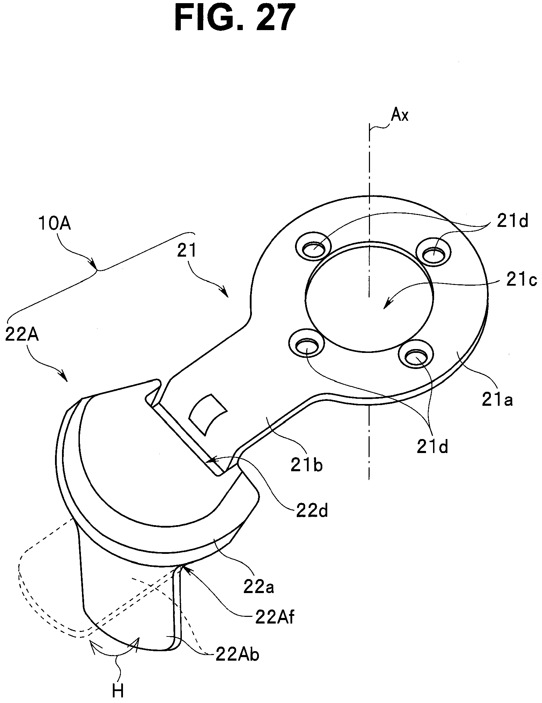

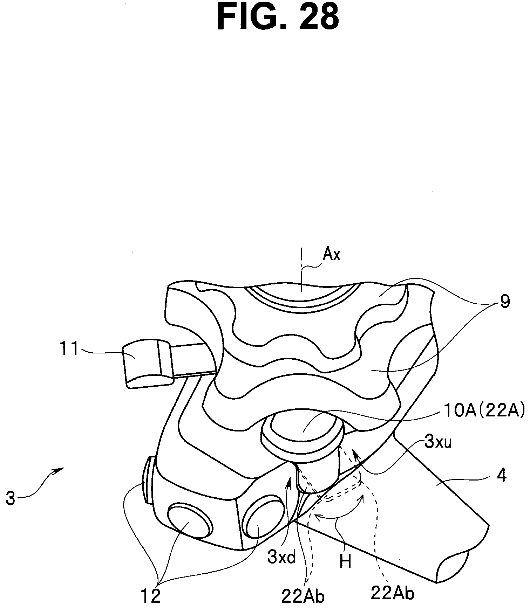

Next, modifications of the first embodiment will be exemplified. First, a first modification will be described below. Fig. 27 and Fig. 28 are diagrams showing the first modification of the endoscope of the present embodiment. Note that the first modification is different from the configuration of the above-described first embodiment merely in terms of the configuration of a forceps lever.

A forceps lever 10A shown in the first modification is similar to the forceps lever 10 of the above-described first embodiment in that the forceps lever 10A is provided to be turnable around the first rotation axis Ax in the operation section 3.

The forceps lever 10A is configured by the lever member 21 and a finger rest member 22A as shown in Fig. 27. Of these members, the lever member 21 has a configuration substantially the same as the configuration of the lever member 21 of the above-described first embodiment. Therefore, detailed description of the lever member 21 is omitted.

The finger rest member 22A is configured to have the main body portion 22a, a projection 22Ab, and the like. The main body portion 22a is an area fixed to the cover attachment portion 21g of the lever member 21. The main body portion 22a has a configuration similar to the configuration of the main body portion 22a of the above-described first embodiment.

The projection 22Ab is formed into a thin plate shape protruding from the neighborhood of the outer periphery portion on the distal end side of the main body portion 22a in the second direction parallel to the direction along the first rotation axis Ax. Herein, the projection 22Ab is coupled to the main body portion 22a through a hinge 22Af that holds the projection 22Ab and the main body portion 22a to be openable/closable with respect to each other. Thus, the projection 22Ab has a configuration that may turn and move in directions of arrows H shown in Fig. 27 and Fig. 28 by action of the hinge 22Af.

In this case, the hinge 22Af has a function of moving the projection 22Ab between a normal position and an open position. Herein, the normal position of the projection 22Ab is a position at which the projection 22Ab is in the form protruding in the second direction parallel to the direction along the first rotation axis Ax. The open position of the projection 22Ab is a position at which the projection 22Ab is in the form protruding in the direction substantially perpendicular to the direction along the first rotation axis Ax. Note that the hinge 22Af includes a click stop mechanism for maintaining the open position (open state) of the projection 22Ab. Although illustration is omitted, a conventionally well-known mechanism is applied to the click stop mechanism in this case.

With such a configuration, the projection 22Ab in the finger rest member 22A of the forceps lever 10A in the first modification is disposed to be openable/closable with respect to the main body portion 22a through the hinge 22Af. Herein, a state of the projection 22Ab indicated by a solid line in Fig. 27 and Fig. 28 is equivalent to the first state. At this time, the projection 22Ab is located at the normal position. In this first state, the projection 22Ab comes into contact with the first wall surface (3xu, 3xd) which is the stopper of the operation section 3. This restricts the forceps lever 10A from turning.

A state of the projection 22Ab indicated by a dotted line in Fig. 27 and Fig. 28 is equivalent to the second state. At this time, the projection 22Ab is in the state moved into the open position with respect to the main body portion 22a by action of the hinge 22Af. At this time, the projection 22Ab does not come into contact with the first wall surface (3xu, 3xd) which is the stopper of the operation section 3. Therefore, the turning range of the forceps lever 10A is enlarged. The remaining configuration is substantially the same as the configuration of the above-described first embodiment.

As described above, the forceps lever 10A according to the first modification can be easily deformed between the first state and the second state since the projection 22Ab in the finger rest member 22 is configured to be openable/closable through the hinge 22Af.

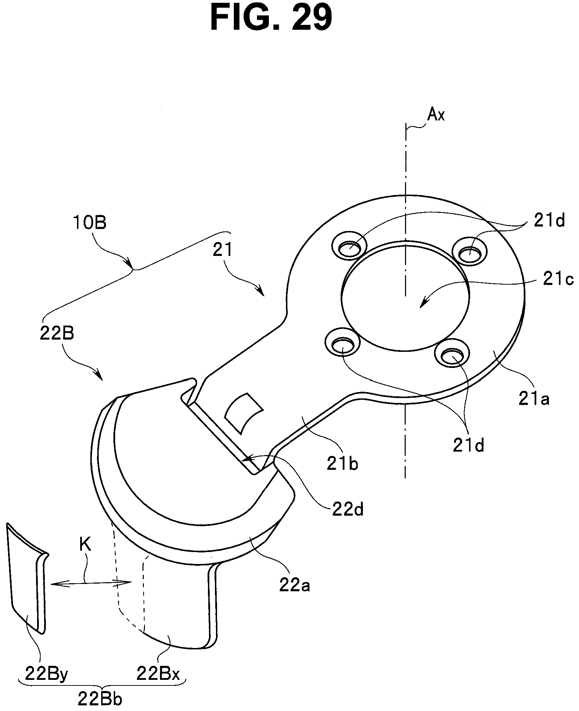

Next, a second modification will be described below. Fig. 29 is a diagram showing the second modification of the endoscope of the present embodiment. Note that the second modification is also different from the configuration of the above-described first embodiment merely in terms of the configuration of a forceps lever.

A forceps lever 10B shown in the second modification is similar to the forceps lever 10 of the above-described first embodiment in that the forceps lever 10B is provided to be turnable around the first rotation axis Ax in the operation section 3.

The forceps lever 10B is configured by the lever member 21 and a finger rest member 22B as shown in Fig. 29. Of these members, the lever member 21 has a configuration substantially the same as the configuration of the lever member 21 of the above-described first embodiment. Therefore, detailed description of the lever member 21 is omitted.

The finger rest member 22B is configured to have the main body portion 22a, a projection 22Bb, and the like. The main body portion 22a is an area fixed to the cover attachment portion 21g of the lever member 21. The main body portion 22a has a configuration similar to the configuration of the main body portion 22a of the above-described first embodiment.

The projection 22Bb is formed into a thin plate shape protruding from the neighborhood of the outer periphery portion on the distal end side of the main body portion 22a in the second direction parallel to the direction along the first rotation axis Ax. Herein, the projection 22Bb is formed by two projection members (22Bx, 22By) formed next to each other along the outer periphery portion of the main body portion 22a. One of the two projection members (22Bx, 22By) is configured to be removable from the main body portion 22a.

In the example shown in Fig. 29, a member denoted by a reference numeral 22By is removable. In this case, a state of the projection 22Bb in which the two projection members (22Bx, 22By) are mounted on the main body portion 22a is equivalent to the first state. The state of the projection 22Bb at this time is the normal state. At this time, the member 22By of the projection 22Bb comes into contact with the first wall surface (3xu, 3xd) which is the stopper of the operation section 3, so that the forceps lever 10B is restricted from turning.

A state of the projection 22Bb in which the member 22By which is one of the two projection members (22Bx, 22By) is detached (the state shown in Fig. 29) is equivalent to the second state. At this time, when turning toward the first wall surface 3xd, the forceps lever 10B turns until one of the projection members, 22Bx, of the projection 22Bb comes into contact with the first wall surface 3xd. Therefore, the turning range of the forceps lever 10B is enlarged by a range corresponding to the member 22By having been detached. The remaining configuration is substantially the same as the configuration of the above-described first embodiment.

As described above, the second modification also enables the forceps lever 10B to be easily deformed between the first state and the second state.

Note that in the second modification, only the member 22By of the projection 22Bb is configured to be removable, but the second modification is not limited to the above configuration, and another form may be considered. For example, the projection member 22Bx which is one of the two projection members (22Bx, 22By) of the projection 22Bb may be configured to be removable. Alternatively, the two projection members (22Bx, 22By) may each be configured to be removable. As a removable configuration, a recess and a projection by which the member 22By can be inserted into the main body portion 22a or the member 22Bx may be formed, or the members 22Bx and 22By may be formed into shapes to be engaged with each other. Alternatively, an electromagnetic force by a magnet or the like may be used.

Instead of the configuration of the above-described second modification (that is, the configuration in which one of the two projection members (22Bx, 22By) of the projection 22Bb is removable from the main body portion 22a), a configuration may be adopted in which at least one (or both) of the two projection members (22Bx, 22By) has an open/close structure using a hinge similar to the hinge of the above-described first modification, or the like. Also in the case of such a configuration, effects similar to the effects of the second modification can be obtained.

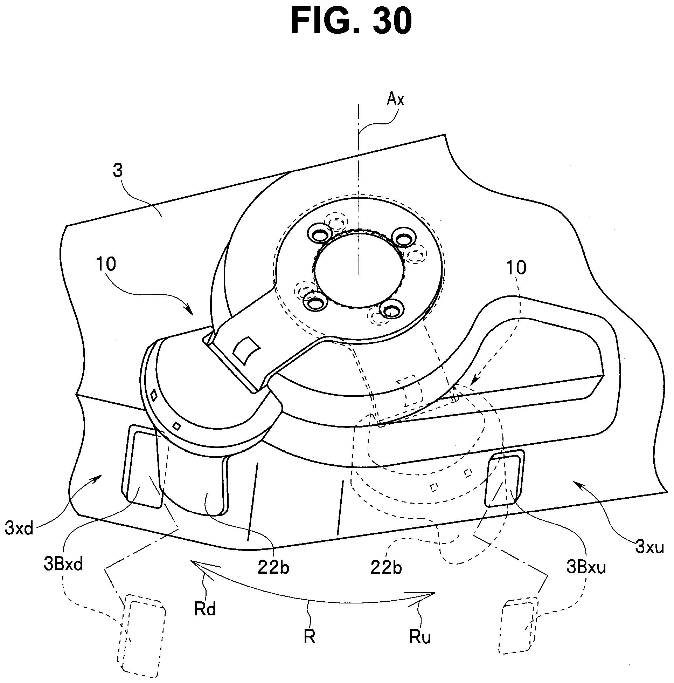

Next, a third modification will be described below. Fig. 30 is a diagram showing the third modification of the endoscope of the present embodiment. The third modification is slightly different from the configuration of the above-described first embodiment in terms of the configuration of stoppers provided in the operation section.

The forceps lever 10 shown in the third modification is similar to the forceps lever 10 of the above-described first embodiment in that the forceps lever 10 is provided to be turnable around the first rotation axis Ax in the operation section 3.

A basic configuration of the operation section 3 is also similar to the basic configuration of the operation section 3 of the above-described first embodiment. The operation section 3 shown in the third modification is different in that the operation section 3 includes stopper blocks (3Bxd, 3Bxu) serving as second stoppers configured to be removable at predetermined positions on the first wall surfaces (3xd, 3xu) as shown in Fig. 30.

Herein, the predetermined positions on the first wall surfaces (3xd, 3xu) are equivalent to positions at which the projection 22b of the finger rest member 22 comes into contact when the forceps lever 10 turns around the first rotation axis Ax. In this case, the predetermined positions include two places, a position on the first wall surface 3xd and a position on the first wall surface 3xu. At the respective predetermined positions, the stopper blocks (3Bxd, 3Bxu) are respectively provided to be removable. The remaining configuration is substantially the same as the configuration of the above-described first embodiment.

With such a configuration, when the stopper block 3Bxd is mounted on the first wall surface 3xd, for example, the projection 22b of the finger rest member 22 of the forceps lever 10 comes into contact with the stopper block 3Bxd to restrict the forceps lever 10 from turning. The state at this time is equivalent to the first state.

When the stopper block 3Bxd on the first wall surface 3xd is detached, the projection 22b of the finger rest member 22 of the forceps lever 10 comes into contact with the first wall surface 3xd to restrict the forceps lever 10 from turning. The state at this time is equivalent to the second state.

Therefore, in the state in which the stopper block 3Bxd is detached, the turning range of the forceps lever 10 is made larger than in the state in which the stopper block 3Bxd is mounted. As a removable configuration, a recess and a projection by which the stopper block 3Bxd can be inserted into the first wall surface 3xd may be formed, or the stopper block 3Bxd and the first wall surface 3xd may be formed into shapes to be engaged with each other. Alternatively, an electromagnetic force by a magnet or the like may be used.

Similarly, when the stopper block 3Bxu is mounted on the first wall surface 3xu, for example, the projection 22b of the finger rest member 22 of the forceps lever 10 comes into contact with the stopper block 3Bxu to restrict the forceps lever 10 from turning. The state at this time is equivalent to the first state.

When the stopper block 3Bxu on the first wall surface 3xu is detached, the projection 22b of the finger rest member 22 of the forceps lever 10 comes into contact with the first wall surface 3xu to restrict the forceps lever 10 from turning. The state at this time is equivalent to the second state. Therefore, in the state in which the stopper block 3Bxu is detached, the turning range of the forceps lever 10 is made larger than in the state in which the stopper block 3Bxu is mounted.

As described above, the third modification also enables the turning range of the forceps lever 10 to be easily switched.

Note that although the third modification exemplifies the configuration example in which the two stopper blocks (3Bxd, 3Bxu) as the second stoppers are provided, the third modification is not limited only to the above form. As another form, a configuration may be adopted in which either one of the two stopper blocks (3Bxd, 3Bxu) is provided, for example.

In the configuration of the above-described third modification (that is, the configuration in which the stopper blocks (3Bxd, 3Bxu) are removable from the first wall surfaces (3xd, 3xu) of the operation section 3), a configuration may further be adopted in which recesses that may allow the projection 22b to avoid contact may be formed at the respective predetermined positions on the first wall surfaces (3xd, 3xu) of the operation section 3. In this case, the stopper blocks (3Bxd, 3Bxu) in the form of covering the respective recesses may be configured to be removable from the recesses. Also in the case of such a configuration, effects similar to the effects of the third modification can be obtained.

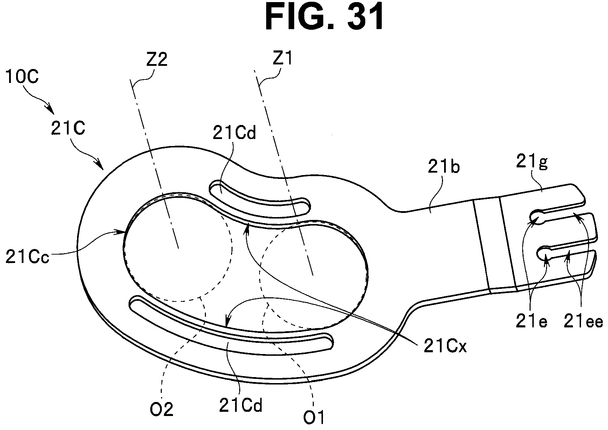

Next, an endoscope of a second embodiment of the present invention will be described below with reference to Fig. 31 to Fig. 34. Note that a basic configuration of the endoscope of the present embodiment is substantially the same as the basic configuration of the endoscope of the above-described first embodiment. The present embodiment is different merely in terms of the configuration of a forceps lever. Therefore, in the following description, constituent members the same as the constituent members of the above-described first embodiment are denoted by the same reference characters, and description of the constituent members will be omitted. Only different portions will be described in detail.

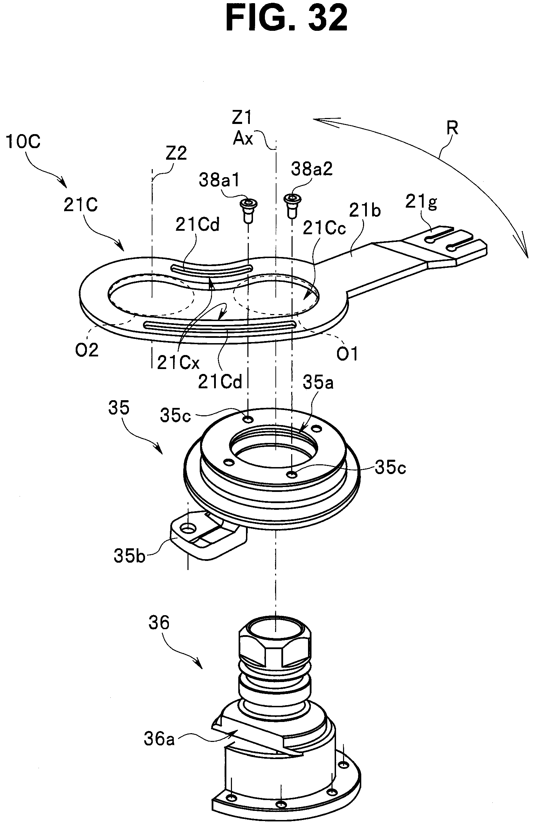

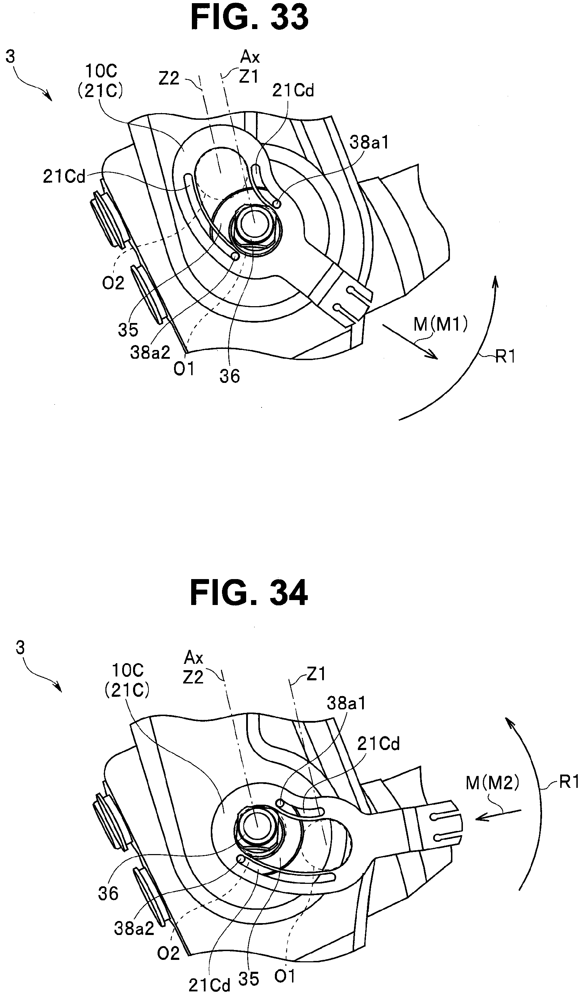

Fig. 31 is a perspective view showing only a lever member of a forceps lever of the endoscope of the second embodiment of the present invention having been taken out. Fig. 32 is an essential part exploded perspective view showing the forceps lever in Fig. 31 and some constituent members (the frame shaft and the link main body) in the driving mechanism for the forceps lever having been taken out. Note that Fig. 32 is equivalent to Fig. 14 in the above-described first embodiment. Fig. 33 and Fig. 34 are diagrams showing an action of the forceps lever in the endoscope of the present embodiment. Of these diagrams, Fig. 33 shows the first state of the forceps lever. Fig. 34 shows the second state of the forceps lever.

A forceps lever 10C in the present embodiment is configured by a lever member 21C and a finger rest member. Note that in Fig. 31 to Fig. 34, illustration of the finger rest member is omitted in order to avoid complicating the drawings. The finger rest member of the forceps lever 10C in the present embodiment has a configuration similar to the configuration of the finger rest member of the above-described first embodiment.

The lever member 21C of the forceps lever 10C in the present embodiment has a shaft through-hole 21Cc in a form formed by joining a first hole O1 (which is a virtual hole) and a second hole O2 (which is a virtual hole) into a substantially arc shape as shown in Fig. 31. The shaft through-hole 21Cc is equivalent to the shaft insertion hole 21c of the forceps lever 10 in the above-described first embodiment. In other words, the shaft through-hole 21Cc is an area that functions as a bearing that allows insertion of the frame shaft 36 and holds the forceps lever 10C to be turnable around the first rotation axis Ax.

Note that a reference character Z1 in Fig. 31 indicates a central axis of the first hole O1. A reference character Z2 in Fig. 31 indicates a central axis of the second hole O2. Reference characters 21Cx in Fig. 31 indicate coupling arc portions that join the first hole O1 and the second hole O2.

Further, the lever member 21C has screw through-slots 21Cd in respective outer edge portions of the two coupling arc portions 21Cx of the shaft through-hole 21Cc. The screw through-slots 21Cd are formed into an arc shape along the respective coupling arc portions 21Cx. The screw through-slots 21Cd are equivalent to the plurality of screw holes 21d of the forceps lever 10 in the above-described first embodiment. At the same time, the screw through-slots 21Cd function as guide slots when the lever member 21C moves as will be described later. The remaining configuration of the lever member 21C is similar to the configuration of the lever member 21 of the above-described first embodiment. In Fig. 32, the configuration of the link main body 35 and the frame shaft 36 is similar to the configuration of the link main body 35 and the frame shaft 36 of the above-described first embodiment.

The following describes an action of the forceps lever 10C in the present embodiment thus configured. First, the forceps lever 10C is incorporated into the operation section 3 in the normal use state as shown in Fig. 32 and Fig. 33.

In other words, in the normal use state (the first state), the forceps lever 10C (the lever member 21C) is incorporated into the link main body 35 and the frame shaft 36 in a state in which the central axis Z1 of the first hole O1 coincides with the first rotation axis Ax, as shown in Fig. 32 and Fig. 33. Thus, the forceps lever 10C (the lever member 21C) is held to be turnable around the first rotation axis Ax (the directions of the arrows R in Fig. 32) on the central axis Z1. At this time, the lever member 21C is screwed to the screw holes 35c of the link main body 35 with guiding stepped screws 38a1 and 38a2 at respective ends on one side of the two screw through-slots 21Cd.

In other words, the lever member 21C and the link main body 35 are fastened to each other with a clearance provided using the guiding stepped screws 38, respectively, through the two screw through-slots 21Cd. Thus, the lever member 21C is configured to be always movable with respect to the link main body 35 in the directions of the arrows R shown in Fig. 32 and directions of arrows M (M1 and M2 directions) shown in Fig. 33 and Fig. 34.

Herein, the screw indicated by the reference character 38a1 extends through the screw through-slot 21Cd disposed on the inner side of the lever member 21C. The screw indicated by the reference character 38a2 extends through the screw through-slot 21Cd disposed on the outer side of the lever member 21C.

At this time, when the lever member 21C rotates in the direction of an arrow R1 shown in Fig. 33 upon receipt of an operation input, the lever member 21C rotates the link main body 35 with the guiding stepped screw 38a1 acting on the one end of the inner one of the screw through-slots 21Cd.

In the above-described state (the state in Fig. 32), the forceps lever 10C (the lever member 21C) is pulled in the direction of an arrow M1 in Fig. 33. Then, the forceps lever 10C (the lever member 21C) is brought into a state (the second state) in which the central axis Z2 of the second hole O2 coincides with the first rotation axis Ax as shown in Fig. 34.

At this time, when the lever member 21C rotates in the direction of the arrow R1 shown in Fig. 34 upon receipt of an operation input, the lever member 21C rotates the link main body 35 with the guiding stepped screw 38a2 acting on the other end of the outer one of the screw through-slots 21Cd.

Note that aside from the configuration through use of the guiding stepped screws, the lever member 21C and the link main body 35 may be fastened with slightly loosened screws. As a further different configuration, a configuration may be adopted in which the lever member 21C and the link main body 35 are fastened using fastening screws of a normal form instead of the guiding stepped screws, for example, and when moving the lever member 21C in the directions of the arrows M shown in Fig. 33 and Fig. 34, the fastening screws are loosened each time.

Thus, the forceps lever 10C (the lever member 21C) is held to be turnable around the first rotation axis Ax on the central axis Z2. At this time, the lever member 21C is screwed to the link main body 35 at ends on the other side of the two screw through-slots 21Cd.

As described above, the above-described second embodiment has the configuration of displacing the forceps lever 10C between the state (the first state) in which the central axis Z1 of the first hole O1 coincides with the first rotation axis Ax and the state (the second state) in which the central axis Z2 of the second hole O2 coincides with the first rotation axis Ax.

This enables the forceps lever 10C to easily switch between two modes of the first state in which the finger rest member (not shown) comes into contact with the stopper of the operation section 3 and the second state in which the finger rest member (not shown) can be turned further without coming into contact with the stopper of the operation section 3.

When in the second state (the state in Fig. 34), a distal end position (the position of the finger rest member) of the lever member 21C is set at a longer distance from the first rotation axis Ax than the distal end position (the position of the finger rest member) of the lever member 21C when in the first state (the state in Fig. 33). This can contribute to improvement in operability when turning the forceps lever 10C (the lever member 21C) when the second state is brought about.

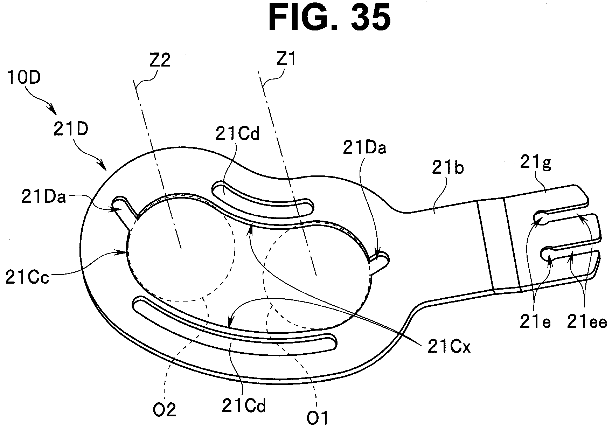

Next, a modification of the second embodiment of the present invention will be described below with reference to Fig. 35 to Fig. 38. Note that a basic configuration of the present modification is substantially the same as the configuration of the above-described second embodiment. The present modification is slightly different in terms of an attachment structure of a lever member of a forceps lever and a link main body. Therefore, in the following description, constituent members the same as the constituent members of the above-described second embodiment are denoted by the same reference characters, and description of the constituent members will be omitted. Only different portions will be described in detail.

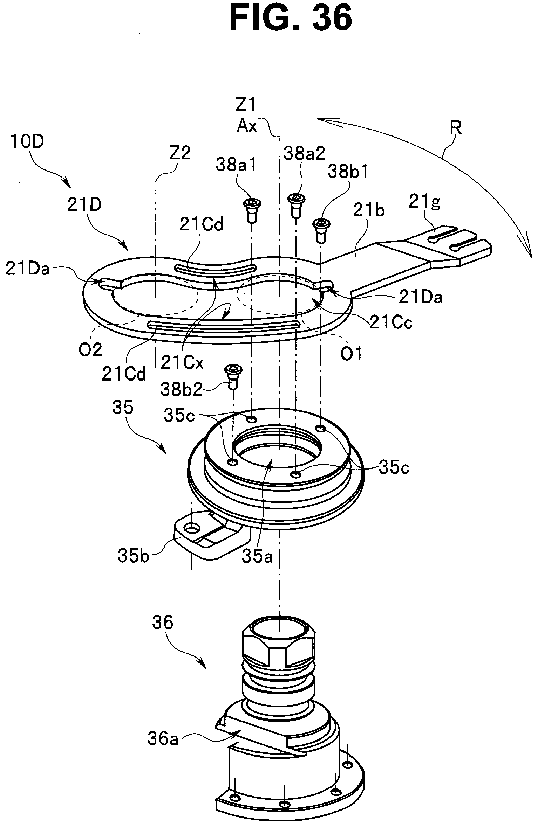

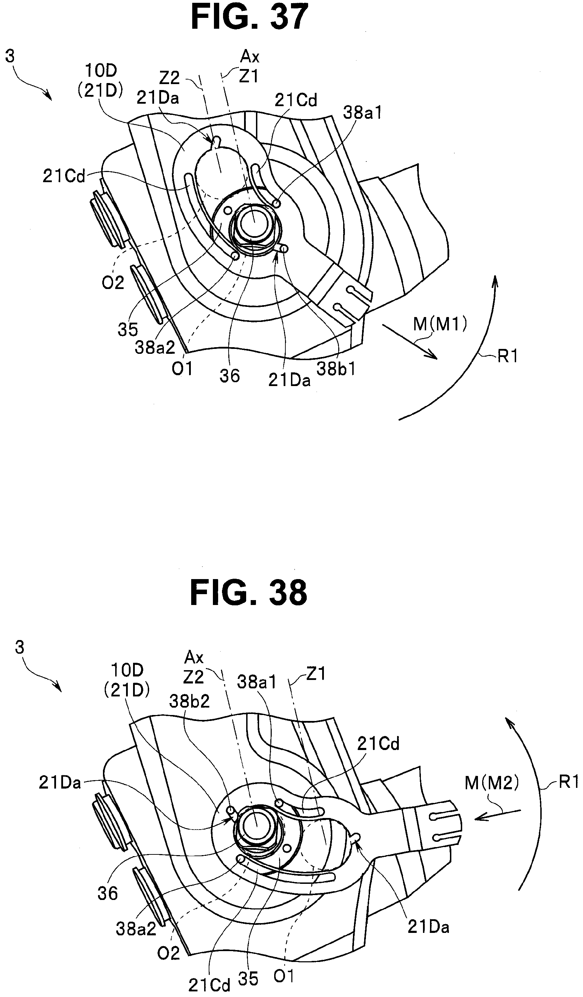

Fig. 35 is a perspective view showing only a lever member of a forceps lever of the modification of the second embodiment of the present invention having been taken out. Fig. 36 is an essential part exploded perspective view showing the forceps lever in Fig. 35 and some constituent members (the frame shaft and the link main body) in a driving mechanism for the forceps lever having been taken out. Fig. 37 and Fig. 38 are diagrams showing an action of the forceps lever in the present modification. Of these diagrams, Fig. 37 shows the first state of the forceps lever. Fig. 38 shows the second state of the forceps lever.

A forceps lever 10D in the present modification is configured by a lever member 21D and a finger rest member. In Fig. 35 to Fig. 38, illustration of the finger rest member is omitted in order to avoid complicating the drawings. The finger rest member of the forceps lever 10D in the present modification has a configuration similar to the configuration of the finger rest member of the above-described first embodiment.

The lever member 21D of the forceps lever 10D in the present modification is similar to the lever member 21C of the above-described second embodiment in that the lever member 21D has the shaft through-hole 21Cc in the form formed by joining the first hole O1 and the second hole O2 into a substantially arc shape as shown in Fig. 35. The shaft through-hole 21Cc functions as a bearing that allows insertion of the frame shaft 36 and holds the forceps lever 10D to be turnable around the first rotation axis Ax.

The lever member 21D is fixed to the link main body 35 to be movable in directions along the arrows M in Fig. 37 and Fig. 38 using a plurality of stepped screws (the guiding stepped screws 38a1, 38a2 and second stepped screws 38b1, 38b2). At the same time, the lever member 21D and the link main body 35 are configured to be turnable in the directions of the arrows R in Fig. 36 with respect to the frame shaft 36.

In further detail, the lever member 21D has two screw through-slots 21Cd of the same form as the screw through-slots 21Cd of the above-described second embodiment. The two screw through-slots 21Cd respectively allow insertion of the guiding stepped screws 38a1 and 38a2 as shown in Fig. 36. The guiding stepped screws 38a1 and 38a2 are screwed with the respectively corresponding screw holes 35c of the link main body 35. Thus, the lever member 21D is fixed at predetermined positions of the link main body 35. In this case, the guiding stepped screws 38a1 and 38a2 function as guide shafts when the lever member 21D moves between the first state and the second state.

The lever member 21D further has two second screw through-slots 21Da. The two second screw through-slots 21Da are formed in a form obtained by notching portions (two locations) at predetermined positions of an inner periphery portion of the shaft through-hole 21Cc. Herein, the predetermined positions of the inner periphery portion of the shaft through-hole 21Cc are regions different from the regions in which the above-described two screw through-slots 21Cd are provided.

Specifically, one of the two second screw through-slots 21Da is formed at a position where the second stepped screw 38b1 is arranged when the lever member 21D is in the first state (see Fig. 37). The other one of the two second screw through-slots 21Da is formed at a position where the second stepped screw 38b2 is arranged when the lever member 21D is in the second state (see Fig. 38). The remaining configuration of the lever member 21D is similar to the configuration of the lever member 21C of the above-described second embodiment. In Fig. 36, the configuration of the link main body 35 and the frame shaft 36 is similar to the configuration of the link main body 35 and the frame shaft 36 of the above-described second embodiment.

The following describes an action of the forceps lever 10D of the present modification thus configured. First, the forceps lever 10D is incorporated into the operation section 3 in the normal use state as shown in Fig. 36 and Fig. 37.

In other words, in the normal use state (the first state), the forceps lever 10D (the lever member 21D) is incorporated into the link main body 35 and the frame shaft 36 in a state in which the central axis Z1 of the first hole O1 coincides with the first rotation axis Ax as shown in Fig. 36 and Fig. 37. Thus, the forceps lever 10D (the lever member 21D) is held to be turnable around the first rotation axis Ax (the directions of the arrows R in Fig. 36) on the central axis Z1.

At this time, the lever member 21D is screwed to the link main body 35 at respective ends on one side of the two screw through-slots 21Cd with the guiding stepped screws 38a1 and 38a2. At the same time, one of the two second screw through-slots 21Da is screwed to the link main body 35 with the second stepped screw 38b1.

In other words, at this time, the lever member 21D and the link main body 35 are fastened with a clearance provided at three points of the two screw through-slots 21Cd and one of the second screw through-slots 21Da using the guiding stepped screws 38a1 and 38a2 and the second stepped screw 38b1. Thus, the lever member 21D is configured to be always movable with respect to the link main body 35 in the directions of the arrows R shown in Fig. 36 and the directions of the arrows M (the M1 and M2 directions) shown in Figs. 37 and 38.

When in the first state shown in Fig. 37 and the lever member 21D rotates in the direction of the arrow R1 shown in Fig. 37 upon receipt of an operation input, the lever member 21D rotates the link main body 35 with the guiding stepped screw 38a1 acting on one end of the inner one of the screw through-slots 21Cd and at the same time with the second stepped screw 38b1 acting on one of the second screw through-slots 21Da.

In the above-described state (the first state in Fig. 37), the forceps lever 10D (the lever member 21D) is pulled in the direction of the arrow M1 in Fig. 37. Then, the forceps lever 10D (the lever member 21D) is brought into the state (the second state) in which the central axis Z2 of the second hole O2 coincides with the first rotation axis Ax as shown in Fig. 38.

At this time, when the lever member 21D rotates in the direction of the arrow R1 shown in Fig. 38 upon receipt of an operation input, the lever member 21D rotates the link main body 35 with the guiding stepped screw 38a2 acting on the other end of the outer one of the screw through-slots 21Cd and at the same time with the second stepped screw 38b2 acting on the other one of the second screw through-slots 21Da.

As described above, the modification of the above-described second embodiment has the configuration in which when fixing the lever member 21D to the link main body 35, the second screw through-slots 21Da are provided in addition to the two screw through-slots 21Cd, and the lever member 21D is fixed to be movable in a predetermined direction with the guiding stepped screws 38a1, 38a2 and the second stepped screws 38b1, 38b2.

With this configuration, the lever member 21D is configured to be movable between the first state (Fig. 37) and the second state (Fig. 38), and rotation of the lever member 21 in the directions of the arrows R in each of the first and second states can be performed more stably. In other words, when rotating the lever member 21D in the first state, the lever member 21D can be supported at two points of the guiding stepped screw 38a1 or 38a2 and the second stepped screw 38b1. When rotating the lever member 21D in the second state, the lever member 21D can be supported at two points of the guiding stepped screw 38a1 or 38a2 and the second stepped screw 38b2. This can ensure more stable rotation of the lever member 21D than in the configuration of the second embodiment.

Note that the second embodiment has described that the finger rest member of the forceps lever 10C has a configuration similar to the configuration of the finger rest member of the above-described first embodiment. However, the configuration of the finger rest member may be different from the configuration of the finger rest member of the above-described first embodiment. For example, the finger rest member may be configured to be fixed to the forceps lever 10C unlike the configuration which is removable from the forceps lever 10C.

Furthermore, the above second embodiment and the modification thereof exemplify the preferred configuration in which the shaft through-hole 21Cc in the lever member 21C of the forceps lever 10C is formed by joining the two holes (the first hole O1 and the second hole O2), which are virtual holes, into an arc shape.

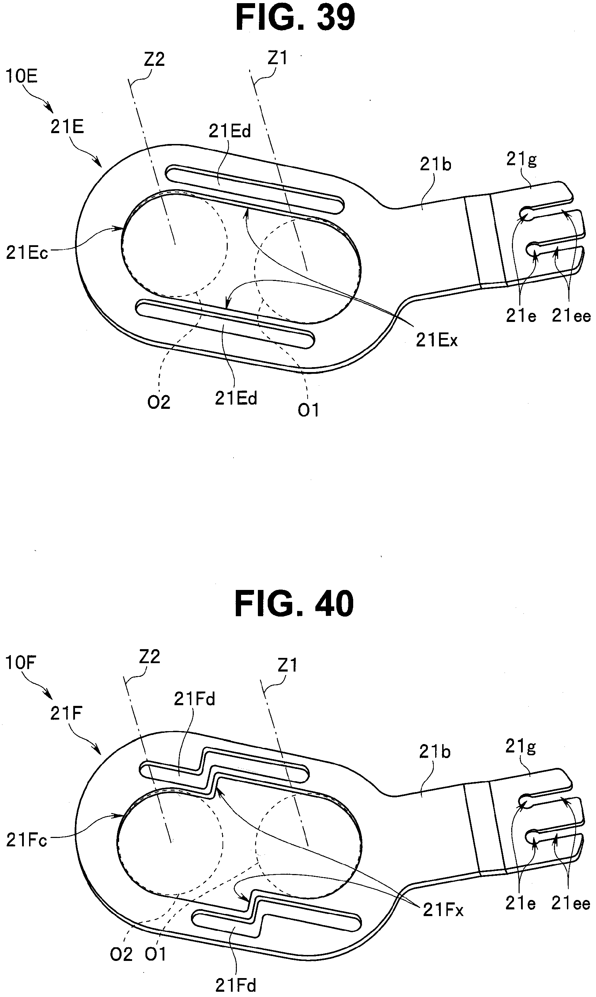

However, the form of the shaft through-hole of the lever member is not limited to the above exemplification. The shaft through-hole may be joining the two holes (the first hole O1 and the second hole O2), which are virtual holes, in a straight line (see a second modification in Fig. 39), or by joining the two holes (O1, O2) as a stepped structure in which a step is provided in the middle of a straight line joining the two holes (O1, O2)(see a third modification in Fig. 40).

Herein, the second modification of the second embodiment of the present invention will be described below with reference to Fig. 39. Fig. 39 is a perspective view showing only a lever member of a forceps lever of the second modification in the endoscope of the second embodiment of the present invention having been taken out.

A basic configuration of the second modification is substantially the same as the configuration of the above-described second embodiment. The second modification is slightly different in terms of a shape of the shaft through-hole in the lever member of the forceps lever. Therefore, in the following description, the same constituent members as those of the above-described second embodiment are denoted by the same reference characters, and description thereof will be omitted. Only different portions will be described below in detail.

The forceps lever 10E of the second modification includes a lever member 21E and a finger rest member (not shown).

As shown in Fig. 39, the lever member 21E includes a shaft through-hole 21Ec formed by joining the first hole O1 and the second hole O2 (which are virtual holes) in a straight line. The shaft through-hole 21Ec functions as a bearing that allows insertion of the frame shaft 36 and holds the forceps lever 10E to be turnable around the first rotation axis Ax.

The lever member 21E has two screw through-slots 21Ed along a straight portion of the shaft through-hole 21Ec. Guiding stepped screws (not shown) are respectively inserted through the two screw through-slots 21Ed. Thus, the lever member 21E is fixed to a predetermined position of the link main body 35, and moves along the screw through-slots 21Ed between the first state and the second state.

Actions of the forceps lever 10E of the present modification thus configured are substantially the same as those of the above-described second embodiment.

The second modification thus configured can provide effects similar to the effects of the second embodiment and the modification thereof. Also, according to the second modification, the shape of the shaft through-hole 21Ec can be simplified more, which contributes to simplification of parts machining.

Next, the third modification of the second embodiment of the present invention will be described below with reference to Fig. 40. Fig. 40 is a perspective view showing only a lever member of a forceps lever of a third modification in the endoscope of the second embodiment of the present invention having been taken out.

A basic configuration of the third modification is substantially the same as the configuration of the above-described second embodiment and the second modification. The third modification is slightly different in terms of a shape of the shaft through-hole in the lever member of the forceps lever. Therefore, in the following description, the same constituent members as those of the above-described second embodiment are denoted by the same reference characters, and description thereof will be omitted. Only different portions will be described in detail.

The forceps lever 10F of the third modification includes a lever member 21F and a finger rest member (not shown).

As shown in Fig. 40, the lever member 21F includes a shaft through-hole 21Fc formed by joining the first hole O1 and the second hole O2 (which are virtual holes) in a substantially straight line, and formed in what is called a stepped structure in which the shaft through-hole 21Fc has steps in the middle position of the outer periphery thereof. The shaft through-hole 21Fc functions as a bearing that allows insertion of the frame shaft 36 and holds the forces lever 10F to be turnable around the first rotation axis Ax.

The lever member 21F has two screw through-slots 21Fd each formed along each of the steps of the shaft through-hole 21Fc. Guiding stepped screws (not shown) are respectively inserted through the two screw through-slots 21Fd. Thus, the lever member 21F is fixed to a predetermined position of the link main body 35, and moves along the screw through-slots 21Fd between the first state and the second state.

Actions of the forceps lever 10F of the present modification thus configured are substantially the same as those of the above-described second embodiment.

The third modification thus configured can provide effects similar to the effects of the second embodiment and the modifications thereof. Also, according to the third modification, by providing the steps in the middle of the shaft through-hole 21Fc, even if an operation error occurs, for example, the lever member 21F is prevented from easily moving to another hole portion. Therefore, the lever member 21F does not move between the first state and the second state contrary to the operator's intention, and can be operated reliably. This can contribute to improved switching operability.

Each of the above-described first and second embodiments exemplifies the configuration example including the forceps raising base 54 as the movable member disposed in the distal end portion 5 of the insertion section 2 of the endoscope 1. However, the movable member is not limited to this exemplification. The movable member disposed in the distal end portion 5 should only be a constituent unit that can move upon receipt of an operational force of the operation lever provided in the operation section 3. As a different configuration example of the movable member, an image pickup apparatus having a mechanism that moves a portion of an image pickup optical system in a direction along an optical axis and a direction along an insertion axis, for example, can be applied.

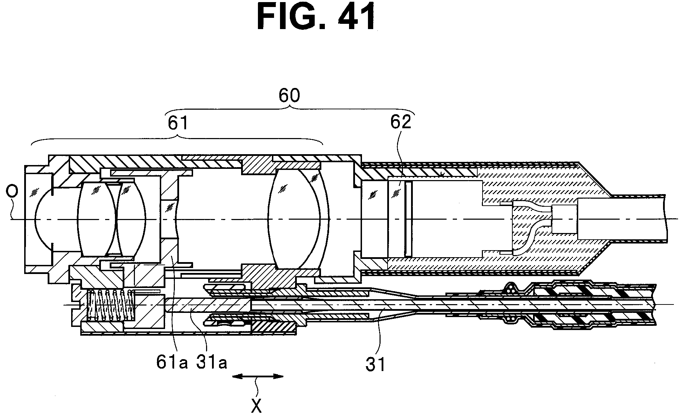

Fig. 41 is a diagram showing a modification of a movable member disposed in the distal end portion of the endoscope of the present invention. In the modification, an image pickup apparatus as a movable member is shown. Fig. 39 is a diagram showing a configuration of the image pickup apparatus by a cross section.

As shown in Fig. 41, an image pickup apparatus 60 is configured by an image pickup optical system 61, an image pickup unit 62, and the like. Herein, the image pickup apparatus 60 itself has a configuration substantially the same as the configuration of an image pickup apparatus having a conventionally common configuration.

In other words, the image pickup optical system 61 is a constituent unit including at least one optical element on which light from a subject is incident. For example, the image pickup optical system 61 is configured by a plurality of optical elements, a plurality of holding members that respectively hold the optical elements, and the like as shown in Fig. 41.

The image pickup unit 62 is composed of an image pickup device, a driving circuit that drives the image pickup device, and an electronic circuit that performs predetermined signal processing and the like upon receipt of an output signal from the image pickup device.

In the image pickup apparatus 60 having such a configuration, a holding member 61a that holds some optical elements of the plurality of optical elements that configure the image pickup optical system 61 is configured to be movable forward and backward in directions (see arrows X in Fig. 41) parallel to a direction along an optical axis O of the image pickup optical system 61. Thus, the holding member 61a is connected integrally with a coupling member 31a to which the distal end of the traction wire 31 is coupled.

Herein, the insertion section and the operation section allow insertion and arrangement of the traction wire 31, and the traction wire 31 has a proximal end coupled to the driving mechanism in the operation section. In this case, the configuration of the insertion section, the operation section, the driving mechanism, and the like is similar to the configurations of the above-described first and second embodiments (see Fig. 1, Fig. 2, Fig. 12, and the like).

With this configuration, the traction wire 31 moves forward and backward in the long axis direction of the insertion section upon receipt of an operation input of the operation lever provided in the operation section. In this case, the operation lever is equivalent to the forceps levers in the above-described first and second embodiments. The operation lever has a configuration similar to the configurations of the forceps levers in the above-described first and second embodiments.

Therefore, in the configuration example in which the image pickup apparatus 60 is applied as the movable member, the long axis direction of the insertion section is equivalent to the first direction. In the case of the configuration example in which the movable member is the image pickup apparatus 60, the finger rest member of the operation lever comes into contact with the stopper (the first wall surface 3xd, 3xu) of the operation section to restrict the holding member 61a from moving to either the proximal end side or the distal end side. The position of the operation lever at this time is equivalent to the first position.

As described above, even in the configuration example in which the image pickup apparatus 60 is applied as the movable member, actions and effects similar to the actions and effects of the above-described first and second embodiments as well as the above-described respective modifications can be obtained.

Note that the configuration of the present invention can be applied to what is called a single-use endoscope in the form to be replaced and utilized each time when at least some constituent members (such as the traction wire 31) among the constituent members of the endoscope are used once as a single-use member. The configuration of the present invention is not limited to the single-use endoscope, but can also be applied to what is called a reusable endoscope in the form to be reutilized upon being subjected to a sterilization and disinfection process, for example, each time when the endoscope is used, for example.

## Claims

1. An endoscope (1) comprising:
an insertion section (2) including a movable member; and
an operation section (3) including a lever (10; 10A; 10B; 10C; 10D; 10E; 10F) and a stopper (3xd, 3xu), and provided on a proximal end side of the insertion section (2), wherein
the movable member is configured to be movable in a first direction,
the lever (10; 10A; 10B; 10C; 10D; 10E; 10F) is held to be turnable around a first rotation axis (Ax), the lever (10; 10A; 10B; 10C; 10D; 10E; 10F) being configured to turn to move the movable member,
the stopper (3xd, 3xu) is configured to come into contact with the lever (10; 10A; 10B; 10C; 10D; 10E; 10F) when the lever (10; 10A; 10B; 10C; 10D; 10E; 10F) is located at a first position,
further,
the lever (10; 10A; 10B; 10C; 10D; 10E; 10F) is configured to be deformed between a first state and a second state,
the first state is a state in which the lever (10; 10A; 10B; 10C; 10D; 10E; 10F) comes into contact with the stopper (3xd, 3xu) at the first position, and
the second state is a state in which the lever (10; 10A; 10B; 10C; 10D; 10E; 10F) does not come into contact with the stopper (3xd, 3xu) at the first position,
**characterized in that**
a distance between the first rotation axis (Ax) and a distal end position of the lever (10; 10A; 10B; 10C; 10D; 10E; 10F) in the second state is longer than the distance between the first rotation axis (Ax) and the distal end position of the lever (10; 10A; 10B; 10C; 10D; 10E; 10F) in the first state.

2. The endoscope (1) according to claim 1, wherein
the lever (10; 10A; 10B; 10C; 10D; 10E; 10F) includes a projection (22b; 22Ab; 22Bb) formed in a second direction parallel to a direction in which the first rotation axis (Ax) extends,
the first state is a state in which the projection (22b; 22Ab; 22Bb) comes into contact with the stopper (3xd, 3xu), and
the second state is a state in which the projection (22b; 22Ab; 22Bb) does not come into contact with the stopper (3xd, 3xu).

3. The endoscope (1) according to any one of claims 1 to 2, wherein
the lever (10C) has a bearing formed by joining a first hole (O1) and a second hole (O2), the first rotation axis (Ax) being inserted into the bearing,
the first state is a state in which the first rotation axis (Ax) is inserted into the first hole (O1) and the lever (10C) is held to be turnable around the first rotation axis (Ax) in the first hole (O1), and
the second state is a state in which the first rotation axis (Ax) is inserted into the second hole (O2) and the lever (10C) is held to be turnable around the first rotation axis (Ax) in the second hole (O2).

4. The endoscope (1) according to claim 3, wherein the bearing is formed by joining the first hole (O1) and the second hole (O2) into an arc shape.

5. The endoscope (1) according to any one of claims 1 to 4, wherein the movable member is a raising base (54) provided in the insertion section (2), the second rotation axis (Ax2) in particular being a rotation axis of the raising base (54).

6. The endoscope (1) according to any one of claims 1 to 5, wherein the movable member is at least one optical element on which light from a subject is incident.

7. The endoscope (1) according to any one of claims 1 to 6, wherein the first position is a position at which the movable member is restricted from moving to the proximal end side; and/or wherein the first position is a position at which the movable member is restricted from moving to a distal end side.

8. The endoscope (1) according to any one of claims 1 to 7, wherein the movable member and the lever are at least partially connected with a single-use member.

9. The endoscope (1) according to any one of claims 1 to 8, wherein
the operation section (3) includes a link mechanism, the link mechanism in particular including a link main body (35), a rod body (34), a coupling member (32), and a coupling member guide (37).

10. The endoscope (1) according to claim 9,
wherein: the rod body (34) has a first end coupled to the link main body (35) and a second end coupled to the coupling member (32); and/or
the coupling member (32) is housed in the coupling member guide (37); and/or
when the link main body (35) turns around the first rotation axis (Ax), the coupling member (32) is configured to slide inside the coupling member guide (37) to move forward and backward in a long axis direction of the insertion section (2).

11. The endoscope (1) according to any one of claims 1 to 10, wherein a portion of the first rotation axis (Ax) has a cross-section formed into a D-shape in a plane perpendicular to the second direction.

## Patentansprüche

1. Endoskop (1) mit:
einem Einführabschnitt (2) mit einem bewegbaren Element; und
einem Bedienabschnitt (3) mit einem Hebel (10; 10A; 10B; 10C; 10D; 10E; 10F) und einem Anschlag (3xd, 3xu), der an einer proximalen Endseite des Einführabschnitts (2) vorgesehen ist, wobei
das bewegbare Element so konfiguriert ist, dass es in einer ersten Richtung bewegbar ist,
der Hebel (10; 10A; 10B; 10C; 10D; 10E; 10F) um eine erste Drehachse (Ax) drehbar gehalten ist, wobei der Hebel (10; 10A; 10B; 10C; 10D; 10E; 10F) so konfiguriert ist, dass er sich dreht, um das bewegbare Element zu bewegen,
der Anschlag (3xd, 3xu) so konfiguriert ist, dass er mit dem Hebel (10; 10A; 10B; 10C; 10D; 10E; 10F) in Kontakt kommt, wenn sich der Hebel (10; 10A; 10B; 10C; 10D; 10E; 10F) in einer ersten Position befindet,
wobei ferner
der Hebel (10; 10A; 10B; 10C; 10D; 10E; 10F) so konfiguriert ist, dass er zwischen einem ersten Zustand und einem zweiten Zustand verformt werden kann,
wobei der erste Zustand ein Zustand ist, in dem der Hebel (10; 10A; 10B; 10C; 10D; 10E; 10F) in der ersten Position mit dem Anschlag (3xd, 3xu) in Kontakt kommt, und
der zweite Zustand ein Zustand ist, in dem der Hebel (10; 10A; 10B; 10C; 10D; 10E; 10F) an der ersten Position nicht mit dem Anschlag (3xd, 3xu) in Kontakt kommt,
**dadurch gekennzeichnet, dass**
ein Abstand zwischen der ersten Drehachse (Ax) und einer distalen Endposition des Hebels (10; 10A; 10B; 10C; 10D; 10E; 10F) im zweiten Zustand länger ist als der Abstand zwischen der ersten Drehachse (Ax) und der distalen Endposition des Hebels (10; 10A; 10B; 10C; 10D; 10E; 10F) im ersten Zustand.

2. Endoskop (1) gemäß Anspruch 1, wobei
der Hebel (10; 10A; 10B; 10C; 10D; 10E; 10F) einen Vorsprung (22b; 22Ab; 22Bb) aufweist, der in einer zweiten Richtung parallel zu einer Richtung ausgebildet ist, in der sich die erste Drehachse (Ax) erstreckt,
der erste Zustand ein Zustand ist, in dem der Vorsprung (22b; 22Ab; 22Bb) mit dem Anschlag (3xd, 3xu) in Kontakt kommt, und
der zweite Zustand ein Zustand ist, in dem der Vorsprung (22b; 22Ab; 22Bb) nicht mit dem Anschlag (3xd, 3xu) in Kontakt kommt.

3. Endoskop (1) gemäß einem der Ansprüche 1 bis 2, wobei
der Hebel (10C) ein Lager aufweist, das durch Verbinden eines ersten Lochs (O1) und eines zweiten Lochs (O2) gebildet ist, wobei die erste Drehachse (Ax) in das Lager eingeführt ist,
der erste Zustand ein Zustand ist, in dem die erste Drehachse (Ax) in das erste Loch (O1) eingeführt ist und der Hebel (10C) so gehalten wird, dass er um die erste Drehachse (Ax) in dem ersten Loch (O1) drehbar ist, und
der zweite Zustand ein Zustand ist, in dem die erste Drehachse (Ax) in das zweite Loch (O2) eingeführt ist und der Hebel (10C) so gehalten wird, dass er um die erste Drehachse (Ax) in dem zweiten Loch (O2) drehbar ist.

4. Endoskop (1) gemäß Anspruch 3, wobei das Lager durch Verbinden des ersten Lochs (O1) und des zweiten Lochs (O2) zu einer Bogenform gebildet ist.

5. Endoskop (1) gemäß einem der Ansprüche 1 bis 4, wobei das bewegbare Element eine in dem Einführabschnitt (2) vorgesehene Hebebasis (54) ist, wobei insbesondere die zweite Drehachse (Ax2) eine Drehachse der Hebebasis (54) ist.

6. Endoskop (1) gemäß einem der Ansprüche 1 bis 5, wobei das bewegbare Element mindestens ein optisches Element ist, auf das Licht von einem Objekt auftrifft.

7. Endoskop (1) gemäß einem der Ansprüche 1 bis 6, wobei die erste Position eine Position ist, an der das bewegbare Element daran gehindert ist, sich zu der proximalen Endseite zu bewegen; und/oder wobei die erste Position eine Position ist, an der das bewegbare Element daran gehindert ist, sich zu der distalen Endseite zu bewegen.

8. Endoskop (1) gemäß einem der Ansprüche 1 bis 7, wobei das bewegbare Element und der Hebel zumindest teilweise mit einem Einwegelement verbunden sind.

9. Endoskop (1) gemäß einem der Ansprüche 1 bis 8, wobei
der Bedienabschnitt (3) einen Verbindungsmechanismus umfasst, wobei der Verbindungsmechanismus insbesondere einen Verbindungshauptkörper (35), einen Stabkörper (34), ein Kopplungselement (32) und eine Kopplungselementführung (37) umfasst.

10. Endoskop (1) gemäß Anspruch 9,
wobei der Stabkörper (34) ein erstes Ende, das mit dem Verbindungshauptkörper (35) verbunden ist, und ein zweites Ende, das mit dem Kupplungselement (32) verbunden ist, aufweist; und/oder
das Kopplungselement (32) in der Kopplungselementführung (37) untergebracht ist; und/oder
wenn sich der Verbindungshauptkörper (35) um die erste Drehachse (Ax) dreht, das Kopplungselement (32) so konfiguriert ist, dass es innerhalb der Kopplungselementführung (37) gleitet, um sich in einer Längsachsenrichtung des Einführabschnitts (2) vorwärts und rückwärts zu bewegen.

11. Endoskop (1) gemäß einem der Ansprüche 1 bis 10, wobei ein Abschnitt der ersten Drehachse (Ax) einen Querschnitt aufweist, der in einer Ebene senkrecht zu der zweiten Richtung D-förmig ausgebildet ist.

## Revendications

1. Endoscope (1) comprenant:
une section d'insertion (2) comprenant un élément mobile; et
une section de commande (3) comprenant un levier (10; 10A; 10B; 10C; 10D; 10E; 10F) et une butée (3xd, 3xu), et disposée sur un côté d'extrémité proximale de la section d'insertion (2), dans lequel
l'élément mobile est conçu pour se déplacer dans une première direction,
le levier (10; 10A; 10B; 10C; 10D; 10E; 10F) est retenu de manière à pouvoir tourner autour d'un premier axe de rotation (Ax), le levier (10; 10A; 10B; 10C; 10D; 10E; 10F) étant conçu pour tourner afin de déplacer l'élément mobile,
la butée (3xd, 3xu) est conçue pour entrer en contact avec le levier (10; 10A; 10B; 10C; 10D; 10E ; 10F) lorsque le levier (10; 10A; 10B; 10C; 10D; 10E; 10F) est situé dans une première position,
en outre
le levier (10; 10A; 10B; 10C; 10D; 10E; 10F) est conçu pour se déformer passant d'un premier état à un second état,
le premier état est un état dans lequel le levier (10; 10A; 10B; 10C; 10D; 10E; 10F) entre en contact avec la butée (3xd, 3xu) dans la première position, et
le second état est un état dans lequel le levier (10; 10A; 10B; 10C; 10D; 10E; 10F) n'entre pas en contact avec la butée (3xd, 3xu) dans la première position,
**caractérisé en ce que**
la distance entre le premier axe de rotation (Ax) et une position d'extrémité distale du levier (10; 10A; 10B; 10C; 10D; 10E; 10F) dans le deuxième état est plus longue que la distance entre le premier axe de rotation (Ax) et la position d'extrémité distale du levier (10; 10A; 10B; 10C; 10D; 10E; 10F) dans le premier état.

2. Endoscope (1) selon la revendication 1, dans lequel
le levier (10; 10A; 10B; 10C; 10D; 10E; 10F) comprend une saillie (22b; 22Ab; 22Bb) formée dans une deuxième direction parallèle à la direction dans laquelle s'étend le premier axe de rotation (Ax),
le premier état est un état dans lequel la saillie (22b; 22Ab; 22Bb) entre en contact avec la butée (3xd, 3xu), et
le deuxième état est un état dans lequel la saillie (22b; 22Ab; 22Bb) n'entre pas en contact avec la butée (3xd, 3xu).

3. Endoscope (1) selon l'une quelconque des revendications 1 à 2, dans lequel
le levier (10C) comporte une partie d'appui formée en reliant un premier trou (O1) et un deuxième trou (O2), le premier axe de rotation (Ax) étant inséré dans l'appui,
le premier état est un état dans lequel le premier axe de rotation (Ax) est inséré dans le premier trou (O1) et le levier (10C) est retenu de manière à pouvoir tourner autour du premier axe de rotation (Ax) dans le premier trou (O1), et
le deuxième état est un état dans lequel le premier axe de rotation (Ax) est inséré dans le deuxième trou (O2) et le levier (10C) est retenu de manière à pouvoir tourner autour du premier axe de rotation (Ax) dans le deuxième trou (O2).

4. Endoscope (1) selon la revendication 3, dans lequel l'appui est formé en reliant le premier trou (O1) et le deuxième trou (O2) en forme d'arc.

5. Endoscope (1) selon l'une quelconque des revendications 1 à 4, dans lequel l'élément mobile est une base relevable (54) disposée dans la section d'insertion (2), le deuxième axe de rotation (Ax2) étant en particulier un axe de rotation de la base relevable (54).

6. Endoscope (1) selon l'une quelconque des revendications 1 à 5, dans lequel l'élément mobile est au moins un élément optique sur lequel la lumière provenant d'un sujet est incidente.

7. Endoscope (1) selon l'une quelconque des revendications 1 à 6, dans lequel la première position est une position dans laquelle le membre mobile ne peut pas se déplacer vers le côté de l'extrémité proximale; et/ou dans laquelle la première position est une position dans laquelle le membre mobile ne peut pas se déplacer vers le côté de l'extrémité distale.

8. Endoscope (1) selon l'une quelconque des revendications 1 à 7, dans lequel l'élément mobile et le levier sont au moins partiellement reliés à un élément à usage unique.

9. Endoscope (1) selon l'une quelconque des revendications 1 à 8, dans lequel
la section de commande (3) comprend un mécanisme de liaison, le mécanisme de liaison comprenant en particulier un corps principal de liaison (35), un corps de tige (34), un élément d'accouplement (32) et un guide d'élément d'accouplement (37).

10. Endoscope (1) selon la revendication 9,
dans lequel: le corps de tige (34) comporte une première extrémité couplée au corps principal de liaison (35) et une seconde extrémité couplée à l'élément d'accouplement (32); et/ou
l'élément d'accouplement (32) est logé dans le guide d'élément d'accouplement (37); et/ou
lorsque le corps principal de liaison (35) tourne autour du premier axe de rotation (Ax), l'élément d'accouplement (32) est conçu de manière à coulisser à l'intérieur du guide d'élément d'accouplement (37) afin de se déplacer vers l'avant et vers l'arrière dans le sens de l'axe longitudinal de la section d'insertion (2).

11. Endoscope (1) selon l'une quelconque des revendications 1 à 10, dans lequel une partie du premier axe de rotation (Ax) présente une section transversale en forme de D dans un plan perpendiculaire à la deuxième direction.
